# EUROPEAN PATENT SPECIFICATION

(11) **EP 2 999 471 B1**
(45) Date of publication and mention of the grant of the patent: **16.12.2020**
(21) Application number: 14800815.4
(22) Date of filing: 19.05.2014
(51) Int. Cl.: A61K 31/495, A61K 31/525, A61K 33/26, A61K 33/40, A61K 41/00, A61P 27/02, A61P 27/10, A61N 5/06, A61K 45/06, A61K 31/121, A61K 31/122, A61K 31/47, A61K 31/498, A61K 31/519

(54) **SYSTEMS, METHODS, AND COMPOSITIONS FOR CROSS-LINKING**
VERNETZUNGSSYSTEME, -VERFAHREN UND -ZUSAMMENSETZUNGEN
SYSTÈMES, PROCÉDÉS ET COMPOSITIONS PERMETTANT UNE RÉTICULATION

(30) Priority: 19.05.2013 US 201361825072 P; 24.10.2013 US 201361895008 P; 12.01.2014 US 201461926340 P; 16.04.2014 US 201461980535 P
(43) Date of publication of application: 30.03.2016
(73) Proprietor: Avedro, Inc., Waltham, MA 02451 (US)
(72) Inventor: KAMAEV, Pavel, Lexington, Massachusetts 02421 (US); FRIEDMAN, Marc, Needham, Massachusetts 02494 (US); SHERR, Evan, Ashland, Massachusetts 01721 (US); MULLER, David, Boston, Massachusetts 02111 (US)
(74) Representative: Mewburn Ellis LLP
(86) International application number: PCT/US2014/038637
(87) International publication number: WO 2014/189849

(56) References cited:
- WO-A2-2009/114513
- WO-A2-2012/158991
- US-A- 5 219 895
- US-A1- 2010 159 029
- US-A1- 2010 203 103
- US-A1- 2011 152 219
- MUN YHUNG JUNG ET AL: "Photoinduced Generation of 2,3-Butanedione from Riboflavin", JOURNAL OF AGRICULTURAL AND FOOD CHEMISTRY, vol. 55, no. 1, 1 January 2007 (2007-01-01), pages 170-174, XP055329005, US ISSN: 0021-8561, DOI: 10.1021/jf061999y
- TZENG D: "PRODUCTION OF HYDROXYL RADICALS IN PHOTODYNAMIC ACTION OF METHIONINE RIBOFLAVIN MIXTURE A CONSEQUENCE OF IRON CATALYZED HABER-WEISS REACTION", BOTANICAL BULLETIN OF ACADEMIA SINICA., vol. 30, no. 3, 1 January 1989 (1989-01-01), pages 171-178, XP55327913, TW ISSN: 0006-8063
- KENNETH V CHACE ET AL: "Effect of oxygen free radicals on corneal collagen", FREE RADICAL RESEARCH COMMUNICAT, HARWOOD ACADEMIC PUBLISHERS, LANGHORNE, PA, US, vol. 12-13, no. Pt. 2, 1 January 1991 (1991-01-01), pages 591-594, XP008182565, ISSN: 8755-0199, DOI: 10.3109/10715769109145834 [retrieved on 2009-07-07]
- CHACE, KV. ET AL.: 'The role of nonenzymatic glycosylation, transition metals, and free radicals in the formation of collagen aggregates.' ARCH BIOCHEM BIOPHYS. vol. 288, no. 2, 01 August 1991, pages 473 - 480, XP024759068
- BURKE, JM. ET AL.: 'Retinal proliferation in response to vitreous hemoglobin or iron.' INVEST OPHTHALMOL VIS SCI. vol. 20, no. 5, May 1981, pages 582 - 92, XP055295664
- KAMAEV, P. ET AL.: 'Photochemical kinetics of corneal cross-linking with riboflavin.' INVEST OPHTHALMOL VIS SCI. vol. 53, no. 4, 05 April 2012, pages 2360 - 2367, XP055248371
- SUN, G.J. ET AL.: 'Properties of 2,3-butanedione and 1-phenyl-1,2-propanedione as new photosensitizers for visible light cured dental resin composites.' POLYMER vol. 41, no. 16, 2000, pages 6205 - 6212, XP004195889

## Description

### CROSS REFERENCE TO RELATED APPLICATIONS

This application claims priority to: U.S. Provisional Patent Application No. 61/825,072, filed May 19, 2013, U.S. Provisional Patent Application No. 61,895,008, filed October 24, 2013, U.S. Provisional Patent Application No. 61/926,340, filed January 12, 2014, and U.S. Provisional Patent Application No. 61/980,535, filed April 16, 2014.

### FIELD OF THE INVENTION

The present invention relates generally to treatment of eye disorders, and more particularly, to systems, methods, and compositions that generate cross-linking activity for treatment of eye disorders.

### BACKGROUND

A variety of eye disorders, such as myopia, keratoconus, and hyperopia, involve abnormal shaping of the cornea. Laser-assisted in-situ keratomileusis (LASIK), for example, is one of a number of corrective treatments that reshape the cornea so that light traveling through the cornea is properly focused onto the retina located in the back of the eye. The success of a particular treatment in addressing abnormal shaping of the cornea depends on the stability of the changes in the corneal structure after the treatment has been applied.

Although treatments may initially achieve desired reshaping of the cornea, the desired effects of reshaping the cornea may be mitigated or reversed at least partially if the collagen fibrils within the cornea continue to change after the desired reshaping has been achieved. To strengthen and stabilize the structure of the cornea after reshaping, some treatments may also initiate cross-linking activity in the corneal tissue. For example, a photosensitizing agent (*e.g*., riboflavin) is applied to the cornea as a cross-linking agent. Once the cross-linking agent has been applied to the cornea, the cross-linking agent is activated by a light source (*e.g*., ultraviolet (UV) light) to cause the cross-linking agent to absorb enough energy to cause the release of free oxygen radicals (*e.g*., singlet oxygen) and/or other radicals within the cornea. Once released, the radicals form covalent bonds between corneal collagen fibrils and thereby cause the corneal collagen fibrils to cross-link and strengthen and stabilize the structure of the cornea.

Due to the advantageous structural changes caused by the cross-linking agent, the cross-linking agent may be applied as the primary aspect of some treatments. For example, a cross-linking agent may be applied to treat keratoconus.

US 2011/0152219 A1 discloses the use of enhancers "with possibly riboflavin" by the method of corneal crosslinking.

### SUMMARY

The present invention is provided according to claim 1.

For example, a composition for applying therapy to a cornea of an eye comprises a cross-linking agent which is riboflavin that generates cross-linking activity in the cornea in response to exposure to a photoactivating light. The composition also comprises at least one additive which is iron. The at least one additive enhances the cross-linking activity generated by the cross-linking agent. In other embodiments, the at least one additive may be iron, e.g., provided by FeSO4.

Also disclosed is a method for applying therapy to a cornea of an eye comprises applying a composition to the cornea, where the composition includes a cross-linking agent that generates cross-linking activity in the cornea in response to exposure to a photoactivating light, and at least one additive different from the cross-linking agent and selected from the group consisting of iron, copper, manganese, chromium, vanadium, aluminum, cobalt, mercury, cadmium, nickel, arsenic, 2,3-butanedione, and folic acid. The method also comprises applying photoactivating light to the cornea to generate cross-linking activity in the cornea. The at least one additive enhances the cross-linking activity generated by the cross-linking agent. In some aspects of the disclosure the cross-linking agent may be selected from the group consisting of riboflavin, 2,3-butanedione, folic acid, quinoxalines, quinolines, dibucaine, Methotrexate, menadione, and derivatives thereof. In one embodiment of the invention the at least one additive may be iron, e.g., provided by FeSO4. In yet other aspects of the disclosure, the at least one additive may be 2,3-butanedione. In further aspects of the disclosure, the at least one additive may be folic acid. In some embodiments, the photoactivating light may be ultraviolet light and/or pulsed (or alternatively continuous). The method may further comprise applying oxygen to the cornea to control the cross-linking activity generated by the cross-linking agent.

In another example, a method for applying therapy to a cornea of an eye, comprises applying a cross-linking agent to the cornea, the cross-linking agent being selected from the group consisting of 2,3-butanedione, folic acid, quinoxalines, quinolines, dibucaine, Methotrexate, menadione, and derivatives thereof. The method also comprises applying photoactivating light to the cornea to generate cross-linking activity in the cornea. In some aspects of the disclosure, the photoactivating light may be ultraviolet light and/or pulsed (or alternatively continuous). The method may further comprise applying oxygen to the cornea to control the cross-linking activity generated by the cross-linking agent.

In yet another example, a method for applying therapy to a cornea of an eye, comprises applying a hydrogen peroxide to the cornea and applying an iron solution to the cornea after applying the hydrogen peroxide. The hydrogen peroxide and iron solution combine to generate cross-linking activity in the cornea.

### BRIEF DESCRIPTION OF THE DRAWINGS

FIG. 1 illustrates a Scheme 1 relating to photochemical transformations of riboflavin under light.
FIG. 2 illustrates a Scheme 2 relating to formation of oxidants by electron transfer reactions and possible initiation of the polymerization by hydrogen abstraction.
FIG. 3 illustrates fluorescence of the corneal digests at 450 nm in relation to the non-cross-linked control (F/Fo) with different concentrations of Iron(II) in solution applied during cross-linking.
FIG. 4 illustrates fluorescence counts of papain digested corneal flaps treated with: (1) dH₂O; (2) H₂O₂; and (3) H₂O₂ and 0.1% Iron(II).
FIG. 5 illustrates fluorescence (relative to untreated controls) recorded at 450 nm for: (1) 0.1% riboflavin (continuous wave (CW)); (2) 0.1% riboflavin + 0.5 mM FeSO₄ (CW); (3) 0.1% riboflavin (pulsed wave (PW) + O₂); and (4) 0.1% riboflavin + 0.5 mM FeSO₄ (PW + O₂).
FIG. 6 illustrates average force vs. displacement of each corneal flap measured by tensiometry for: 0.1% riboflavin (CW) (2) and 0.1% riboflavin + 0.5 mM FeSO₄ (CW) (3), relative to controls (1).
FIGS. 7-8 illustrate, for repeated experiments, average force vs. displacement of each corneal flap measured by tensiometry for 0.1% riboflavin (PW + O₂) (2) and 0.1% riboflavin + 0.5 mM FeSO₄ (PW + O₂) (3), relative to controls (1).
FIG. 9 illustrates a mechanism for the formation of 2,3-butanedione from riboflavin and singlet oxygen.
FIG. 10 illustrates displacement-force diagrams for corneal flaps treated with 1% solution of 2,3-butanedione (BD) in dH₂O without ultraviolet (UV) light (left panel) and with UV light (right panel) (365 nm, 30 mW for 4 min).
FIG. 11 illustrates fluorescence (relative to the untreated controls, Fo) recorded at 450 nm from the papain digested 200 µm-thick corneal flaps, treated with 1% solution of BD with and without UV light.
FIG. 12 illustrates displacement-force diagrams for corneal flaps treated with UV light (365 nm, 30 mW for 4 min) and: 0.1% solution of riboflavin in dH₂O (1); 0.1% BD in dH₂O (4); and mixture of 0.1% riboflavin and 0.1% BD in dH₂O (3), relative to controls (1).
FIG. 13 illustrates fluorescence (relative to the untreated controls, Fo) recorded at 450 nm from the papain digested 200 µm-thick corneal flaps, treated with 30 mW UVA for 4 min and: 0.1% solution of riboflavin in dH₂O; 0.1% BD in dH₂O; and mixture of 0.1% riboflavin and 0.1% BD in dH₂O.
FIG. 14 illustrates folic acid (FA).
FIG. 15 illustrates pterine-6-carboxylic acid (PCA), a photoproduct of FA.
FIG. 16 illustrates the absorption spectrum of 0.001% FA in PBS.
FIG. 17 illustrates fluorescence spectrum of 0.001% FA in PBS (excitation 360 nm).
FIG. 18 illustrates absorbance of FA in phosphate buffer at 360 nm.
FIG. 19 illustrates displacement vs. force curves for corneal samples: (1) not exposed to UV light; (2) 0.1% riboflavin, exposed to UV light; (3) 0.1% FA, exposed to UV light; and (4) mixture of 0.1% riboflavin and 0.1% FA, exposed to UV light, with UV exposure of 365 nm, 30 mW/cm², pulsed 1 second on: 1 second off for 8 minutes total, with oxygen ambience over the cornea.
FIG. 20 illustrates fluorescence of the corneal samples after digestion with papain (excitation 360 nm): (1) not exposed to UV light; (2) 0.1% riboflavin, exposed to UV light; (3) 0.1% FA, exposed to UV light; and (4) mixture of 0.1% riboflavin and 0.1% FA, exposed to UV light, with UV exposure at 365 nm, 30 mW/cm², pulsed 1 second on: 1 second off for 8 minutes total, with oxygen ambience over the cornea.
FIG. 21 illustrates displacement vs. force curves for corneal samples (thickness 300 um, 3 samples in each group): (1) controls unexposed to UV light; (2) 0.1% FA in a buffer, exposed UV light; (3) 0.1% riboflavin in buffer saline solution, exposed to UV light; and (4) mixture of 0.1% FA in 0.1% riboflavin in buffer saline solution, exposed to UV light, UV exposure at 365 nm, 30 mW/cm², pulsed 1 second on: 1 second off for 8 minutes total, with oxygen ambience over the cornea.
FIG. 22 illustrates biaxial extensiometry of 200 um thick corneal flaps, soaked with 0.4% Olaquindox in PBS with irradiation for 4 min with 30 mW/cm2 (CW) (2) and irradiation for 8 minutes (1 second on:1 second off) with 30 mW/cm2 of pulsed UVA light and O₂ (3), relative to controls (1).
FIG. 23 illustrates relative fluorescence recorded at 450 nm of the cross-linked flaps with 0.4% Olaquindox in PBS: (1) non-irradiated control; (2) irradiation for 4 min with 30 mW/cm² continuously (CW); and (3): irradiation for 8 minutes (1 second on:1 second off) with 30 mW/cm2 of pulsed UVA light and O₂.
FIG. 24 illustrates alkaline hydrolysis of riboflavin (A) into 1,2-dihydro-6,7-dimethyl-2-keto-1-D-ribityl-3-quinoxaline-carboxylic acid (B).
FIG 25 illustrates UV/Vis spectra of the 0.1% riboflavin-5-phosphate in BBBS kept at 120 °C for different amounts of time.
FIG. 26 illustrates the rate of hydrolysis of riboflavin at 120 °C in BBBS as measured by absorbance at 450 nm (0.1% solution and 0.5% solution, A₀ is the absorbance before heating).
FIG. 27 illustrates UV/Vis spectra which is obtained from FIG. 25 by subtracting absorbance of the remaining riboflavin.
FIG. 28 illustrates spectral analysis of the hydrolyzed solution after 90 min at 120 °C (absorbance of residual riboflavin was subtracted from the analyzed spectrum).
FIG. 29 illustrates change in the absorbance of the different peaks during the time of hydrolysis.
FIG. 30 illustrates the synthesis of the sodium salt of 1,2-Dihydro-6,7-dimethyl-2-keto-1-D-ribityl-3-quinoxalinecarboxylic acid monohydrate 2, an early stage alkaline degradation product of riboflavin.
FIG. 31 illustrates NMR spectrum of the synthesized riboflavin degradation product 2.
FIG. 32 illustrates monophosphorylated riboflavin (5-FMN) in buffered blood bank saline without thermal treatment.
FIG. 33 illustrates 5- FMN in buffered blood bank saline after 1 hour of thermal treatment.
FIG. 34 illustrates 5-FMN in buffered blood bank saline after 2 hours of thermal treatment.
FIG. 35 illustrates 5-FMN in buffered blood bank saline after 3 hours of thermal treatment.
FIG. 36 illustrates 5-FMN in buffered blood bank saline after 4 hours of thermal treatment.
FIG. 37 illustrates HPLC trace of the synthesized riboflavin degradation product 2.
FIG. 38 illustrates absorption spectra of thermally heated (red line) and not heated (blue line) riboflavin solutions (recorded in a quartz cuvette with 200 µm optical path).
FIG. 39 illustrates the Difference between two spectra in FIG. 32.
FIG. 40 illustrates fluorescence of the digested corneas: non-cross-linked control (black line), cross-linked with 0.1% riboflavin which was not heated (blue line), cross-linked with thermally treated riboflavin solution (red line).
FIG. 41 illustrates relative fluorescence of the cross-linked corneal samples: red - using thermally treated riboflavin solution, blue - using not heated 0.1% riboflavin solution.
FIG. 42 illustrates sodium salt of 1,2-Dihydro-6,7-dimethyl-2-keto-1-D-ribityl-3-quinoxalinecarboxylic acid monohydrate.
FIG. 43 illustrates absorbance spectrum of 0.001% solution of sodium salt of 1,2-Dihydro-6,7-dimethyl-2-keto-1-D-ribityl-3-quinoxalinecarboxylic acid monohydrate in BBBS (quartz, 1 cm light path)
FIG. 44 illustrates UV absorbance of sodium salt of 1,2-Dihydro-6,7-dimethyl-2-keto-1-D-ribityl-3-quinoxalinecarboxylic acid monohydrate at 360 nm (solution in BBBS, quartz cuvette with 1 cm light path).
FIG. 45 illustrates fluorescence of sodium salt of 1,2-Dihydro-6,7-dimethyl-2-keto-1-D-ribityl-3-quinoxalinecarboxylic acid monohydrate in BBBS solutions (excitation 360 nm).
FIG. 46 illustrates fluorescence of the digested corneas: non-cross-linked control (black line), cross-linked with 0.1% riboflavin in BBBS (red line), cross-linked with 0.1% sodium salt of 1,2-Dihydro-6,7-dimethyl-2-keto-1-D-ribityl-3-quinoxalinecarboxylic acid monohydrate (blue line).
FIG. 47 illustrates fluorescence of the digested corneal flaps: non-cross-linked control (black line), cross-linked with 0.1% riboflavin in BBBS (red line), cross-linked with 0.1% sodium salt of 1,2-Dihydro-6,7-dimethyl-2-keto-1-D-ribityl-3-quinoxalinecarboxylic acid monohydrate (blue line).
FIG. 48 illustrates 3-hydroxy-2-quinoxalinecarboxylic acid.
FIG. 49 illustrates absorbance spectra of 3-hydroxy-2-quinoxalinecarboxylic acid.
FIG. 50 illustrates fluorescence of 3-hydroxy-2-quinoxalinecarboxylic acid's solutions with different concentrations in BBBS, recorded with excitation of 360 nm.
FIG. 51 illustrates fluorescence of the papain digested corneal flaps (200 µm thick) cross-linked with 0.17% (red lines) and 0.017% (blue lines) solutions of 3-hydroxy-2-quinoxalinecarboxylic acid in BBBS (no epithelium, 20 min soaking time, 30 mW/cm² for 4 min), relative to non-cross-linked controls (black lines).
FIG. 52 illustrates tensiometry plots of 200- µm thick corneal flaps irradiated at 30 mW/cm2 for 4 min, preliminary saturated for 20 min with 0.17% riboflavin (red lines) and 0.17% 3-hydroxy-2-quinoxalinecarboxylic acid (3H2QXCA, green lines)relative to non-cross-linked controls (black lines).
FIG. 53 illustrates relative fluorescence of the papain digested corneal flaps (200 µm thick) cross-linked with 0.1% riboflavin (blue bar, solution 2) and 0.1% riboflavin containing 0.02% solution of 3-hydroxy-2-quinoxalinecarboxylic acid in BBBS (red bar, solution 1) (no epithelium, 20 min soaking time, 30 mW/cm² for 4 min), where F₀ - fluorescence of a non-cross-linked flap.
FIG. 54 illustrates 4-methyl-3-oxo-3,4-dihydro-2-quinoxaline carboxylic acid.
FIG. 55 illustrates absorbance spectrum of 0.01 mg/ml solution of 4-methyl-3-oxo-3,4-dihydro-2-quinoxaline carboxylic acid in BBBS (quartz, 1 cm light path).
FIG. 56 illustrates fluorescence of 4-methyl-3-oxo-3,4-dihydro-2-quinoxaline carboxylic acid in BBBS solutions (excitation 360 nm).
FIG. 57 illustrates fluorescence of the papain-digested corneal flaps: non-cross-linked control (black line), cross-linked with 0.1 mg/ml (red line) and 1 mg/ml (green line) solutions of 4-methyl-3-oxo-3,4-dihydro-2-quinoxaline carboxylic acid in BBBS, where corneas were de-epithelialized, soaked with the solution of the cross-linker for 20 min and then irradiated for 4 min with 30 mW/cm² UVA light (360 nm).
FIG. 58 illustrates quinoxaline (also called benzopyrazine) as a heterocyclic compound containing a ring complex made up of benzene ring and a pyrazine ring.
FIG. 59 illustrate quinoxaline-2,3-dithione cyclic dithio-carbonate (Morestan) and trithiocarbonate (Eradox).
FIG. 60 illustrates Methotrexate.
FIG. 61 illustrates menadione (vitamin K₃),
FIG. 62 provides an block diagram of an example delivery system for deliveringcross-linking agent(s) (optionally combined with additive(s)) and photo-activating light to a cornea to generate cross-linking of corneal collagen within the cornea.

### DESCRIPTION

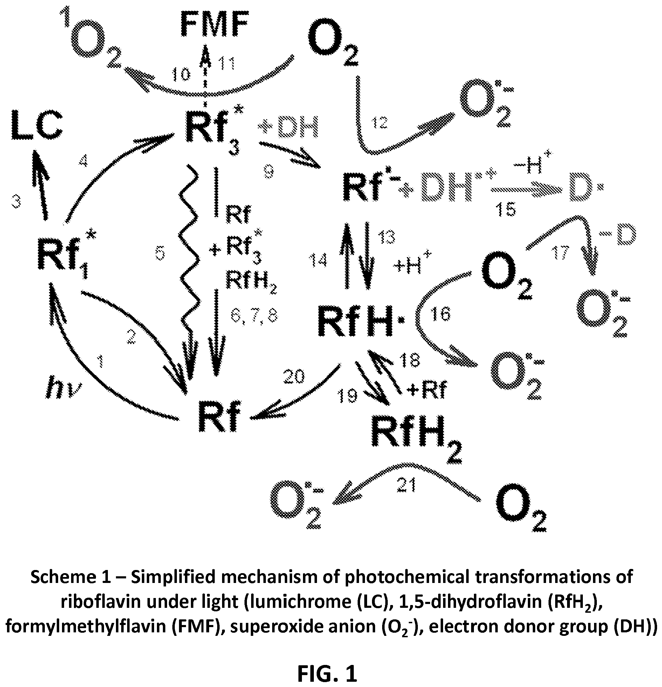

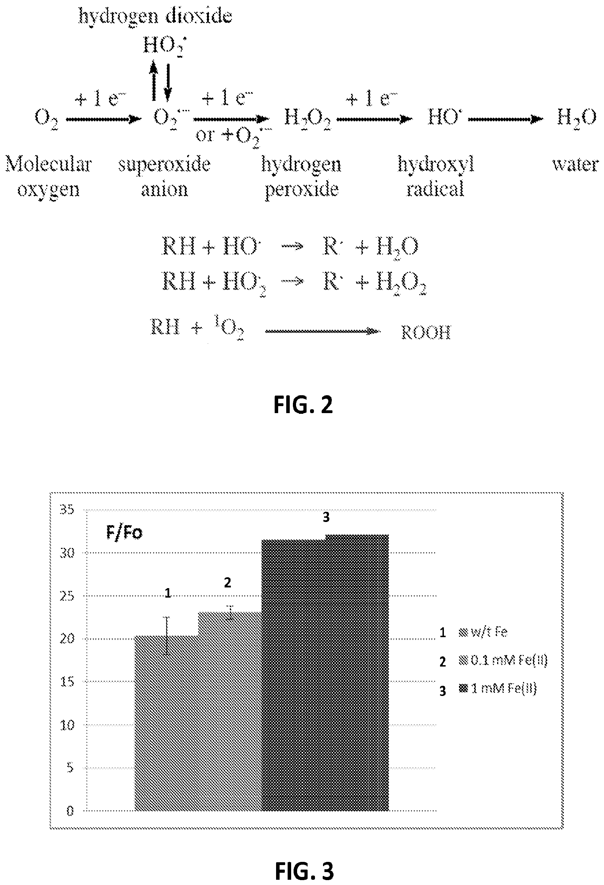

An overall Scheme 1 shown in FIG. 1 summarizes photochemical transformations of riboflavin (Rf) under UV light and its interactions with various donors (DH) via electron transfer. An important practical point is that not all the superoxide anions generated in Scheme 1 are consumed in the overall reaction. Superoxide anions (in equilibrium with its conjugate acid) can produce hydrogen peroxide and subsequently hydroxyl radicals, as shown by Scheme 2 in FIG. 2. In particular, Scheme 2 shows the formation of oxidants by electron transfer reactions and possible initiation of the polymerization by hydrogen abstraction. The overall picture of the action of different reactive oxygen species (ROS) in Scheme 2 on collagen is complex. Superoxide anions are not very reactive, but are able to degrade collagen. As opposite, hydroxyl radicals alone are able to induce protein aggregation. Hydroxyl radicals are considered to be initiators of polymerization not only for proteins. However, a mixture of the hydroxyl radicals with superoxide anions (with excess of the former) stimulates degradation of proteins.

As it is clear from Scheme 2, hydrogen peroxide is the immediate precursor of the hydroxyl radicals. To test the idea that increasing the concentration of hydroxyl radicals leads to acceleration of collagen cross-linking, it would be worthy to accelerate the decomposition of hydrogen peroxide. One way to do so involves employing Fenton's reaction:

H₂O₂ + Fe(II) → OH⁻ + OH˙ + Fe(III)

Concentration of the Fe(II) in solution is not too high because Fe(III) reacts with superoxide anion regenerating Fe(II):

Fe(III) + O₂ ˙⁻ → O₂ + Fe(II)

Moreover, hydroxy-complexes of Fe(III), while irradiated with UV light photo-chemically reduces into Fe(II). Hydroxyl radicals generated during this photo-reduction is an additional bonus:

Fe(III)-OH + (UV light) → Fe(II) + OH˙

Copper ions can be used instead of iron, and it is a promising sign that cross-linking of collagen is observed under this condition.

The addition of traces of metals such as iron or copper to riboflavin formulations enhances corneal collagen cross-linking with UV light. Other metals that may mediate formation of reactive oxygen and nitrogen species include, for example: manganese, chromium, vanadium, aluminum, cobalt, mercury, cadmium, nickel, or arsenic.

### Cross-linking with Ribolfavin and Iron(II)

The following study conducted an investigation to establish how the presence of Iron(II) in riboflavin solution enhances collagen-related fluorescence indicative of cross-linking activity.

### A. Materials and Method

De-epithelialized eyes were soaked for 20 minutes with 0.1% riboflavin in dH₂O only, or 1 mM of FeSO₄ (Iron(II) Sulfate) in 0.1% riboflavin in dH₂O, in an incubator set at 37°C by using a rubber ring to hold the solution on top of the cornea. Corneas were pan-corneally irradiated with a top hat beam (3% root mean square) for 8 minutes (7.2 J total dose) in a cylinder filled with oxygen with 365-nm light source (pulsing 1 second on, 1 second off) (UV LED NCSU033B[T]; Nichia Co., Tokushima, Japan) at the chosen irradiance (30 mW/cm²) which was measured with a power sensor (model PD-300-UV; Ophir, Inc., Jerusalem, Israel) at the corneal surface. Before the start of irradiation, oxygen's exposure was 2 minutes. Corneal flaps (approximately 200 µm thick) were excised from the eyes with aid of Intralase femtosecond laser (Abbot Medical Optics, Santa Ana, CA). The average thickness of the corneal flaps was calculated as a difference between the measurements before and after the excision from the eyes with an ultrasonic Pachymeter (DGH Technology, Exton, PA). The flaps were washed with distilled water two times, dried with filter paper, washed with dH₂O two times, and then dried in a vacuum until the weight change became less than 10% (Rotary vane vacuum pump RV3 A652-01-903, BOC Edwards, West Sussex, UK). Each flap was digested for 2.5 hours at 65°C with 2.5 units/ml of papain (from Papaya latex, Sigma) in 1 ml of papain buffer [BBBS (pH 7.0-7.2), 2 mM L-cysteine and 2 mM EDTA]. Papain digests were centrifuged for 5 seconds at 2200xG (Mini centrifuge 05-090-100, Fisher Scientific), diluted 0.5 times with BBBS and fluorescence of the solutions was measured with excitation of λex = 360 nm in a QM-40 Spectrofluorometer (Photon Technology Int., London, Ontario, Canada). The fluorescence of the papain buffer was taken into account by measuring fluorescence in the absence of tissue and subtracting this value from the fluorescence of the samples.

This method was used because the non-enzymatic cross-link density in collagens was previously quantified with use of papain digest fluorescence. There is a linear relationship between fluorescence and increasing cross-linking activity.

### B. Results/Conclusion

FIG. 3 illustrates the fluorescence of the corneal digests at 450 nm in relation to the non-cross-linked control (F/Fo) with different concentrations of Iron(II) in solution applied during cross-linking. As the results in FIG. 3 show, the presence of Iron(II) in riboflavin solution enhances collagen-related fluorescence at 450 nm after exposure to UVA light, indicative of cross-linking activity.

### Cross-linking with Hydrogen Peroxide and Iron(II) (Reference example)

The following study conducted an investigation to test corneal cross-linking using a 0.1% Iron(II) solution made from FeSO₄ solution with a hydrogen peroxide pre-soak.

### A. Materials and Method

Pig eyes were shipped overnight on ice from an abattoir (SiouxPreme, Sioux City, IA), rinsed in saline. Eyes were several days old at time of experiment. Eyes were cleaned and epithelium was removed. Corneal flaps (approximately 200 µm thick) were excised from the eyes with aid of Intralase femtosecond laser (Abbot Medical Optics, Santa Ana, CA). The average thickness of the corneal flaps was calculated as a difference between the measurements before and after the excision from the eyes with an ultrasonic Pachymeter (DGH Technology, Exton, PA).

Corneal flaps were soaked in either distilled water or diluted H₂O₂ (1%) for 20 minutes. Flaps soaked in H₂O₂ were either rinsed twice with distilled water or removed from H₂O₂ and placed in 0.1% FeSO₄ solution in distilled water for an additional 20 minute soak followed by a 2X rinse with distilled water. Flaps were dried in a vacuum until the weight change became less than 10% (Rotary vane vacuum pump RV3 A652-01-903, BOC Edwards, West Sussex, UK). Each flap was digested for 2.5 hours at 65°C with 2.5 units/ml of papain (from Papaya latex, Sigma) in 1 ml of papain buffer [BBBS (pH 7.0-7.2), 2 mM L-cysteine and 2 mM EDTA]. Papain digests were centrifuged for 5 seconds at 2200xG (Mini centrifuge 05-090-100, Fisher Scientific), diluted 0.5 times with BBBS and fluorescence of the solutions was measured with excitation of λex = 360 nm in a QM-40 Spectrofluorometer (Photon Technology Int., London, Ontario, Canada). The fluorescence of the papain buffer was taken into account by measuring fluorescence in the absence of tissue and subtracting this value from the fluorescence of the samples.

Treatments:
- Control:: Corneal flaps were placed in 1.5 mL Eppendorf tubes and soaked with dH₂O for 20 minutes.
- H₂O₂:: Corneal flaps were placed in 1.5 mL Eppendorf tubes and soaked with 1% of H₂O₂ for 20 minutes. Corneal flaps were rinsed twice with dH₂O before drying.
- H₂O₂ + Iron(II):: Corneal flaps were placed in 1.5 mL Eppendorf tubes and soaked with 1% of H₂O₂ for 20 minutes. Flaps were removed from the original tube and transferred to a new Eppendorf tube with 0.1% Iron(II) solution in dH₂O for 20 minutes. Flaps were rinsed twice with dH₂O before drying.

### B. Results/Conclusion

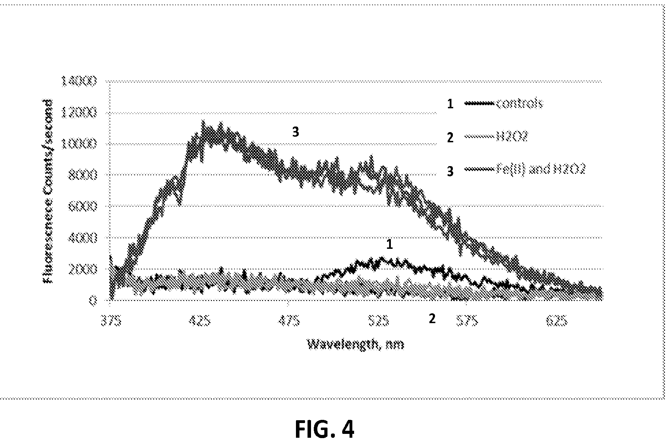

FIG. 4 illustrates fluorescence counts of papain digested corneal flaps treated with: (1) dH₂O; (2) H₂O₂; and (3) H₂O₂ and 0.1% Iron(II). As the results in FIG. 4 show, a fluorescence pattern for the H₂O₂ + 0.1% Iron(II) condition is similar to normal cross-linking patterns. This demonstrates that cross-linking occurs when flaps are placed in the Iron solution after H₂O₂, but not when they were exposed to only H₂O₂.

### Further Cross-linking with Riboflavin and Iron(II)

The following study examined the effects of 0.5 mM FeSO₄ in 0.1% riboflavin in dH₂O on corneal collagen crosslinking. Samples were either irradiated continuously, or with oxygen and pulsed UVA. The following description combines data from two separate days of experiments.

### A. Materials and Methods

Pig eyes were shipped overnight on ice from an abattoir (SiouxPreme, Sioux City, IA), rinsed in saline. Eyes were cleaned and epithelium was removed. Eyes were soaked for 20 minutes with dH₂O, 0.1% riboflavin in dH₂O or 0.5 mM FeSO₄ in 0.1% riboflavin in dH₂O in an incubator set at 37°C by using a rubber ring to hold the solution on top. If specified, eyes were placed in a beaker with a light oxygen stream for 2 minutes in the incubation chamber prior to irradiation. Corneas were pan-corneally irradiated with a top hat beam (3% root mean square) for the chosen time (4 or 8 minutes) with 365-nm light source (UV LED NCSU033B[T]; Nichia Co., Tokushima, Japan) at the chosen irradiance (30 mW/cm², pulsed or non-pulsed) which was measured with a power sensor (model PD-300-UV; Ophir, Inc., Jerusalem, Israel) at the corneal surface. Corneal flaps (approximately 200 µm thick) were excised from the eyes with aid of Intralase femtosecond laser (Abbot Medical Optics, Santa Ana, CA). The average thickness of the corneal flaps was calculated as a difference between the measurements before and after the excision from the eyes with an ultrasonic Pachymeter (DGH Technology, Exton, PA). The flaps were placed into a biaxial extensometer (CellScale Biotester 5000, Waterloo, ON), using biorake attachments with 5 tines spanning a width of 3 mm. Each sample was stretched at a constant rate of 4 µm/s in saline at 37°C until sample failure. The flaps were washed with distilled water 2 times, dried with filter paper, washed with dH₂O two times, and then dried in a vacuum until the weight change became less than 10% (Rotary vane vacuum pump RV3 A652-01-903, BOC Edwards, West Sussex, UK). Each flap was digested for 2.5 hours at 65°C with 2.5 units/ml of papain (from Papaya latex, Sigma) in 1 ml of papain buffer [BBBS (pH 7.0-7.2), 2 mM L-cysteine and 2 mM EDTA]. Papain digests were centrifuged for 5 seconds at 2200xG (Mini centrifuge 05-090-100, Fisher Scientific), diluted 0.5 times with 1XBBBS and fluorescence of the solutions was measured with excitation of λex = 360 nm in a QM-40 Spectrofluorometer (Photon Technology Int., London, Ontario, Canada). The fluorescence of the papain buffer was taken into account by measuring fluorescence in the absence of tissue and subtracting this value from the fluorescence of the samples.

Treatments:
- Control:: After being soaked in dH₂O, corneal flaps were cut at approximately 200 µm.
- 0.1% Riboflavin, CW:: After being soaked in 0.1% riboflavin, eyes were illuminated with 30 mW/cm² of UVA light for 4 minutes, continuous wave (CW). Corneal flaps were cut at approximately 200 µm.
- 0.1% Riboflavin + 0.5 mM FeSO₄, CW:: After being soaked in 0.1% riboflavin + 0.5mM FeSO₄, eyes were illuminated with 30 mW/cm² of UVA light for 4 minutes, continuous wave (CW). Corneal flaps were cut at approximately 200 µm
- 0.1% Riboflavin, PW + O₂:: After being soaked in 0.1% riboflavin, eyes were placed in a beaker with oxygen stream for 2 minutes, then illuminated with 30 mW/cm² of pulsed UVA light for 8 minutes (1 second on:1 second off) (pulsed wave (PW)). Oxygen was supplied continuously to the beaker during the time of exposure. Corneal flaps were cut at approximately 200 µm.
- 0.1% Riboflavin + 0.5 mM FeSO₄, PW + O₂:: After being soaked in 0.1% riboflavin + 0.5mM FeSO₄, eyes were placed in a beaker with oxygen stream for 2 minutes, then illuminated with 30 mW/cm² of pulsed UVA light for 8 minutes (1 second on:1 second off) (pulsed wave (PW)). Oxygen was supplied continuously to the beaker during the time of exposure. Corneal flaps were cut at approximately 200 µm.

### B. Results

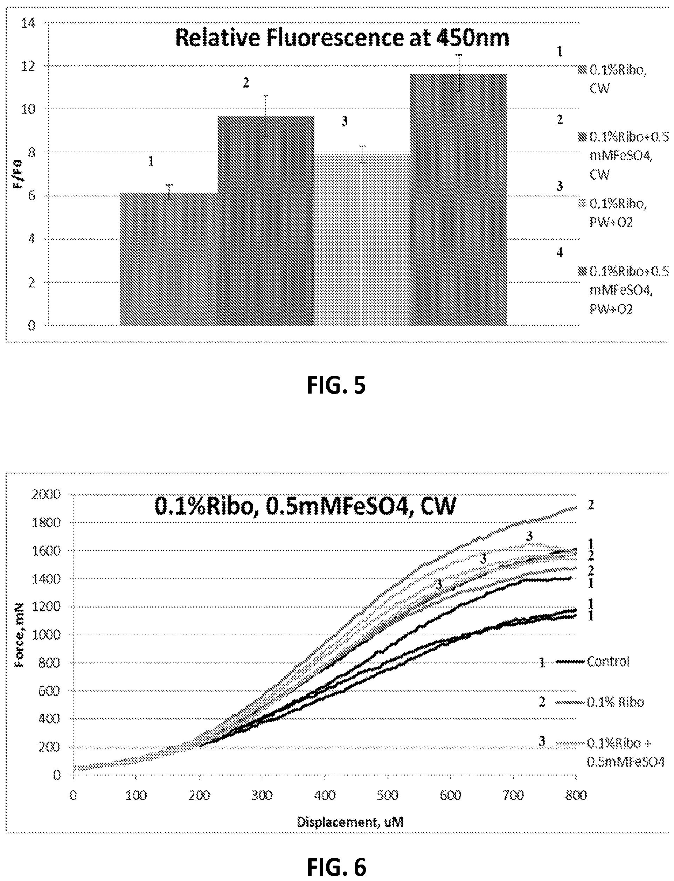

FIG. 5 illustrates fluorescence (relative to untreated controls) recorded at 450 nm for: (1) 0.1% riboflavin (continuous wave (CW)); (2) 0.1% riboflavin + 0.5 mM FeSO₄ (CW); (3) 0.1% riboflavin (pulsed wave (PW) + O₂); and (4) 0.1% riboflavin + 0.5 mM FeSO₄ (PW + O₂).

FIG. 6 illustrates average force vs. displacement of each corneal flap measured by tensiometry for: 0.1% riboflavin (CW) (2) and 0.1% riboflavin + 0.5 mM FeSO₄ (CW) (3), relative to controls (1).

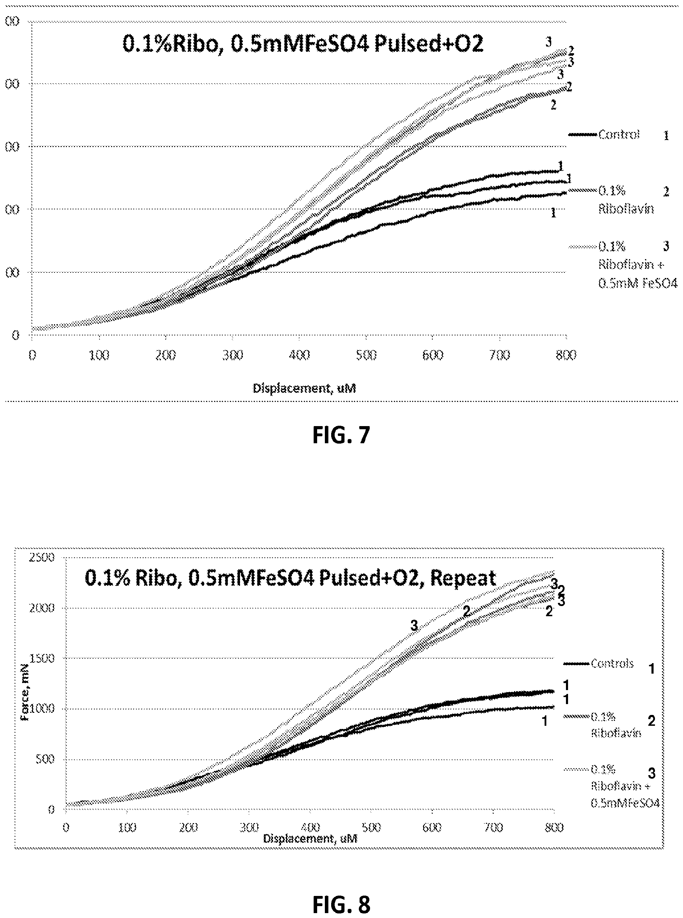

FIGS. 7-8 illustrate, for repeated experiments, average force vs. displacement of each corneal flap measured by tensiometry for 0.1% riboflavin (PW + O₂) (2) and 0.1% riboflavin + 0.5 mM FeSO₄ (PW + O₂) (3), relative to controls (1).

### C. Conclusion

Two methods were used to determine corneal cross-linking in corneal flaps. First, the papain digestion results show an increase in fluorescence under both the continuous (CW) and pulsing (PW) + O₂ condition with the addition of FeSO₄. The second method, tensiometry, only displayed an increase in biaxial tension for the PW + O₂ condition when FeSO₄ was added.

The relative fluorescence graph of FIG. 5 shows an increase in fluorescence counts when FeSO₄ is added to 0.1% riboflavin as well as when UVA application is changed from a continuous dose to a pulsed dose with oxygen.

FIG. 6 shows the tensiometry results from the continuous UVA dose treatment groups. The 0.1% riboflavin group and the 0.1% riboflavin + 0.5 mM FeSO₄ group both display a similar correlation between force and displacement as the displacement increases. Both groups are higher than the control group.

FIGS. 7 and 8 show the tensiometry results from the pulsed UVA application with oxygen treatment groups. The 0.1% riboflavin + 0.5 mM FeSO₄ group shows a slightly greater force as the displacement increases. The increase in force was greater for the PW + O₂ treatment groups than the increase in the CW treatment groups.

### Cross-linking with Riboflavin and 2,3-Butanedione (Reference example)

Diacetyl (2,3-butanedione) is an α-diketone that is present naturally in butter and a variety of foods including dairy products and alcoholic beverages as a product of bacterial fermentation. The U.S. Food and Drug Administration granted diacetyl GRAS (generally recognized as safe) status as a direct food ingredient, and consumption of the low levels of diacetyl present in food has not been reported to present a human health risk.

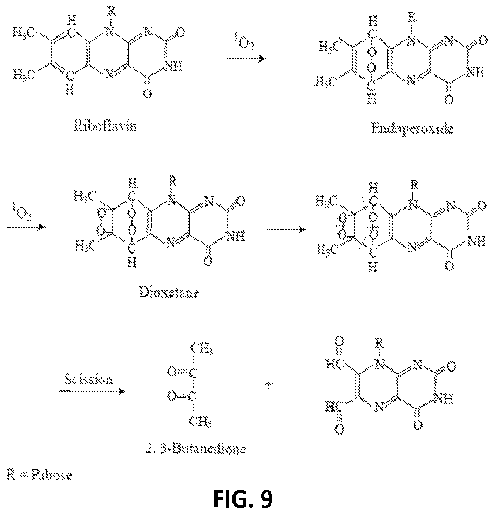

According to studies, 2,3-butanedione is a major volatile product detected in the riboflavin solutions after irradiation with UV light. The mechanism includes the interaction between singlet oxygen and riboflavin. FIG. 9 illustrates the mechanism for the formation of 2,3-butanedione from riboflavin and singlet oxygen. Studies confirm generation of 2,3-butanedione in riboflavin solution after UV irradiation and its participation in corneal cross-linking has been proposed. The following study conducted an investigation to measure quantitatively the cross-linking efficiency of 2,3-butanedione when using it for corneal cross-linking with and without riboflavin.

### A. Materials and Methods

Pig eyes were shipped overnight on ice from an abattoir (SiouxPreme, Sioux City, IA), rinsed in saline. Eyes were cleaned and epithelium was removed. Eyes were soaked for 20 minutes with 0.1% riboflavin in dH₂O, or 0.1% 2,3-butanedione (BD) in dH₂O in an incubator set at 37°C by using a rubber ring to hold the solution on top of the eye. Corneas were pan-corneally irradiated with a top hat beam (3% root mean square) for 4 minutes with 365-nm light source (UV LED NCSU033B[T]; Nichia Co., Tokushima, Japan) at irradiance 30 mW/cm², which was measured with a power sensor (model PD-300-UV; Ophir, Inc., Jerusalem, Israel) at the corneal surface. Corneal flaps (approximately 200 µm thick) were excised from the eyes with aid of Intralase femtosecond laser (Abbott Medical Optics, Santa Ana, CA). The average thickness of the corneal flaps was calculated as a difference between the measurements before and after the excision from the eyes with an ultrasonic Pachymeter (DGH Technology, Exton, PA). The flaps were then placed into a biaxial extensometer (CellScale Biotester 5000, Waterloo, ON), using biorake attachments with 5 tines spanning a width of 3 mm. Each sample was stretched at a constant rate of 4 µm/s in saline at 37°C until sample failure. The flaps were washed with distilled water, dried in a vacuum until the weight change became less than 10% (Rotary vane vacuum pump RV3 A652-01-903, BOC Edwards, West Sussex, UK). Each flap was digested for 2.5 h at 65°C with 2.5 units/ml of papain (from Papaya latex, Sigma) in 1 ml of papain buffer [BBBS (pH 7.0-7.2), 2 mM L-cysteine and 2 mM EDTA]. Papain digests were centrifuged for 5 seconds at 2200xG (Mini centrifuge 05-090-100, Fisher Scientific), diluted 0.5 times with 1XBBBS and fluorescence of the solutions was measured with excitation of λex = 360 nm in a QM-40 Spectrofluorometer (Photon Technology Int., London, Ontario, Canada). The fluorescence of the papain buffer was taken into account by measuring fluorescence in the absence of tissue and subtracting this value from the fluorescence of the samples.

### B. Results/Conclusion

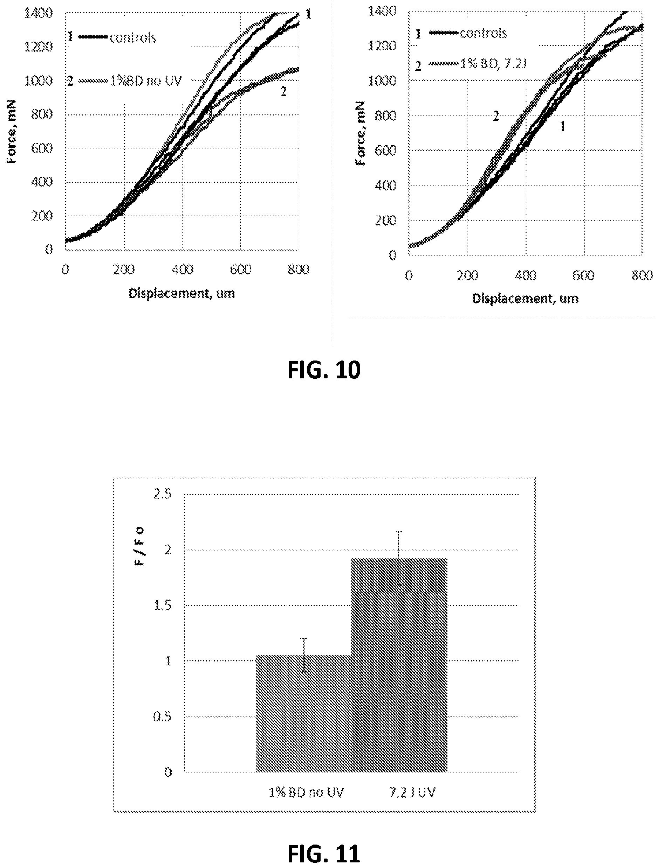

FIG. 10 illustrates displacement-force diagrams for corneal flaps treated with 1% solution of 2,3-butanedione (BD) in dH₂O without ultraviolet (UV) light (left panel) and with UV light (right panel) (365 nm, 30 mW for 4 min).

FIG. 11 illustrates fluorescence (relative to the untreated controls, Fo) recorded at 450 nm from the papain digested 200 µm-thick corneal flaps, treated with 1% solution of BD with and without UV light.

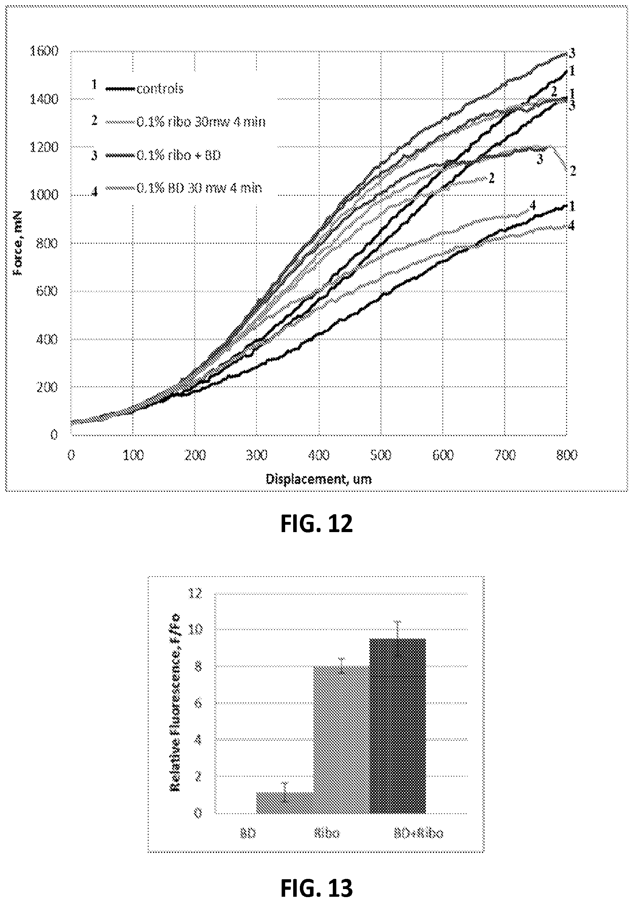

FIG. 12 illustrates displacement-force diagrams for corneal flaps treated with UV light (365 nm, 30 mW for 4 min) and: 0.1% solution of riboflavin in dH₂O (1); 0.1% BD in dH₂O (4); and mixture of 0.1% riboflavin and 0.1% BD in dH₂O (3), relative to controls (1).

FIG. 13 illustrates fluorescence (relative to the untreated controls, Fo) recorded at 450 nm from the papain digested 200 µm-thick corneal flaps, treated with 30 mW UVA for 4 min and: 0.1% solution of riboflavin in dH₂O; 0.1% BD in dH₂O; and mixture of 0.1% riboflavin and 0.1% BD in dH₂O.

As shown in FIGS. 10 and 11, 2,3-butanedione itself (without UV light) does not cross-link corneal flaps, but when irradiated with 365 nm UV light, it leads to the increase of the corneal stiffness and fluorescence output recorded from the treated cornea. FIGS. 12 and 13 show change in the stiffness of the corneal flaps when mixture of BD with riboflavin is used for the cross-linking.

Accordingly, 2,3-butanedione can be used as an additive to a riboflavin formulation to increase cross-linking efficacy.

Based on its participation in corneal cross-linking described above, it is also contemplated that 2,3-butanedione can also be used as a primary cross-linking agent (without riboflavin).

### Cross-linking with Products from Hydrolysis of Riboflavin (Reference example)

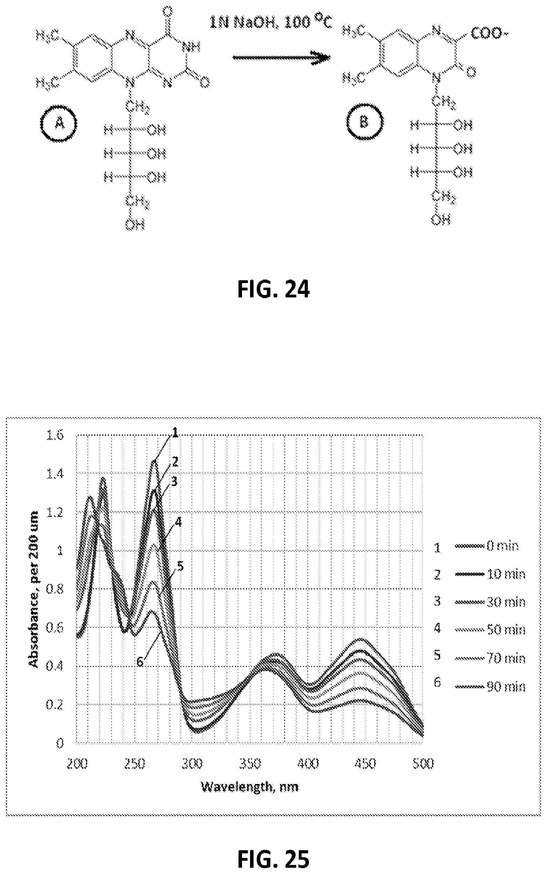

Riboflavin is hydrolyzed in alkaline solution to give urea and 1,2-dihydro-6,7-dimethyl-2-keto-1-D-ribityl-3-quinoxaline-carboxylic acid among the hydrolysis products. FIG. 24 illustrates alkaline hydrolysis of riboflavin (A) into 1,2-dihydro-6,7-dimethyl-2-keto-1-D-ribityl-3-quinoxaline-carboxylic acid (B).

The kinetics of the alkaline degradation has been followed spectrophotometrically and it has been noted that the optical density of the original riboflavin solution decreases at 450 and 370 nm but increases at 310 nm. When the present inventors heated 0.1% riboflavin-5-phosphate solution in 0.85% blood buffered bank saline (BBBS) (Thermo Scientific) at 120 °C, they detected a similar development (as shown in FIG. 25). In particular, FIG 25 illustrates UV/Visible (Vis) light spectra of the 0.1% riboflavin-5-phosphate in BBBS kept at 120 °C for different amounts of time.

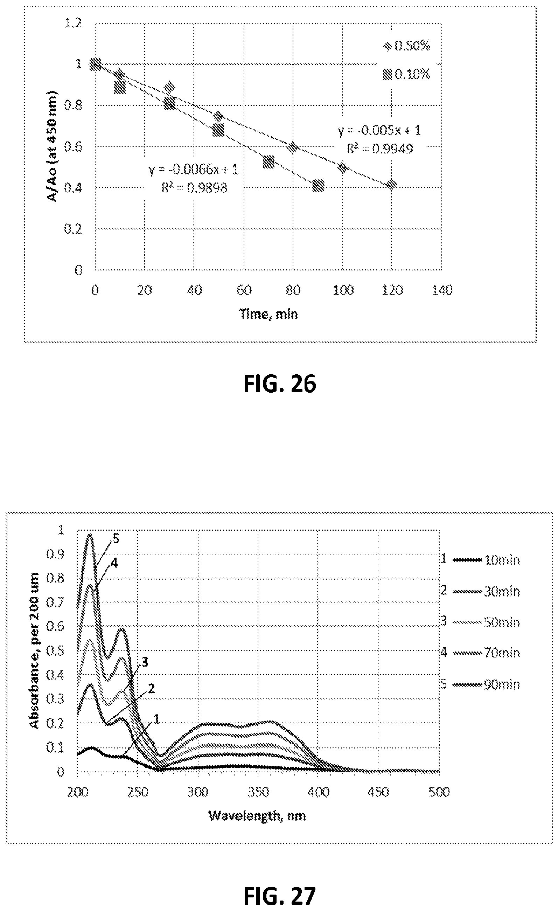

During the heating procedure, concentration of riboflavin decreases with time (FIG. 25, see absorbance change at 450 nm). The rate of destruction of riboflavin depends also on its concentration in solution. For example, the present inventors have found that a 0.1% solution hydrolyzes 1.3 times more quickly than a 0.5% solution (as shown in FIG 26). In particular, FIG. 26 illustrates the rate of hydrolysis of riboflavin at 120 °C in BBBS as measured by absorbance at 450 nm (0.1% solution and 0.5% solution, A₀ is the absorbance before heating).

At the same time, there is an accumulation of products resulting from the hydrolysis (as shown in FIG. 27). In particular, FIG. 27 illustrates UV/Vis spectra which is obtained from FIG. 25 by subtracting absorbance of the remaining riboflavin.

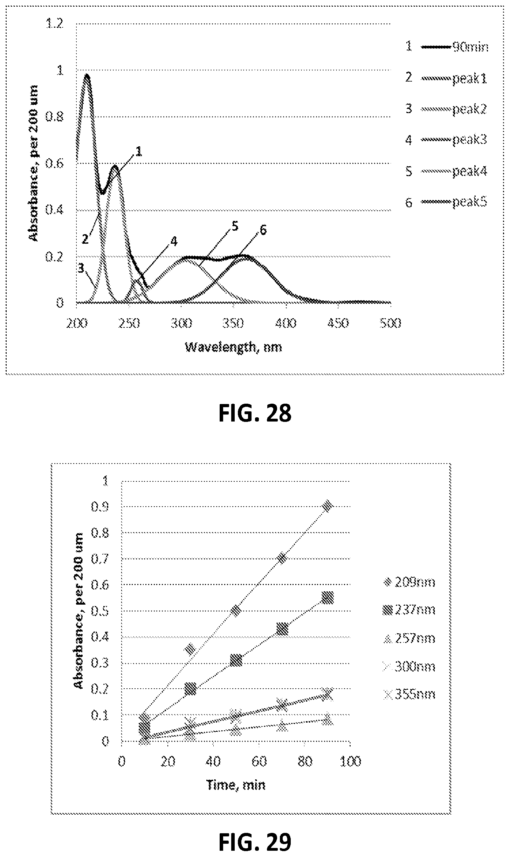

It is possible to analyze spectra from FIG. 27 by combining Gaussian absorption peak shapes (as shown in FIG. 28). In particular, FIG. 28 illustrates spectral analysis of the hydrolyzed solution after 90 min at 120°C (absorbance of residual riboflavin was subtracted from the analyzed spectrum).

Accumulation of the products during the riboflavin hydrolysis follows by the linear increase in absorption at 209, 237, 257, 300, and 355 nm (as shown in FIG. 29). In particular, FIG. 29 illustrates change in the absorbance of the different peaks during the time of hydrolysis.

For HPLC (high-pressure liquid chromatography) analysis of the degradation products of riboflavin, a Dionex UltiMate 3000 with a Lichrospher WP300 RP18 column, 250 mm × 4.0 mm, 5µm from Merck Millipore was used. Mobile phase (A) was water containing monobasic potassium phosphate (7.35 g/L) and (B) methanol. In general, isocratic conditions (15% B, flow of 1.70 mL/min, 30 min, 40 °C) were suitable for analysis of the degradation process. UV spectra were obtained during the HPLC analysis using a diode array detector (λ = 200-450nm) and the chromeleon software.

A procedure with water (A) as mobile phase and acetonitrile (B) with various TFA concentrations was successfully used for final product analysis (vide supra) but failed for the analysis of the degradation process.

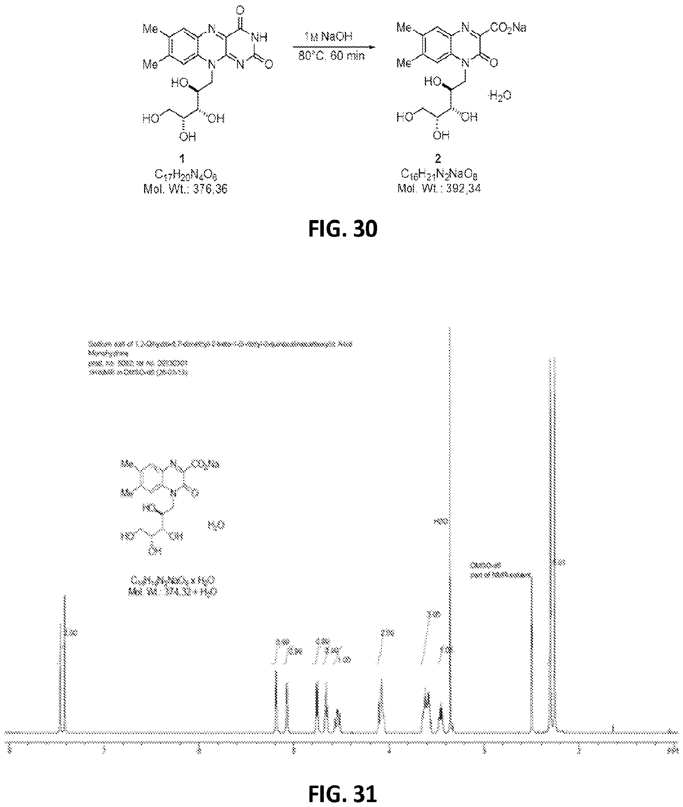

For cross-linking treatments, the sodium salt of 1,2-Dihydro-6,7-dimethyl-2-keto-1-D-ribityl-3-quinoxalinecarboxylic acid monohydrate 2, an early stage alkaline degradation product of riboflavin, as shown in FIG. 30, can be synthesized. The quinoxaline 2 can be prepared by using a synthetic protocol from Surrey et al. J. Am. Chem. Soc. 1951, 73, 3236-2338. FIG. 31 illustrates NMR spectrum of the synthesized riboflavin degradation product 2.

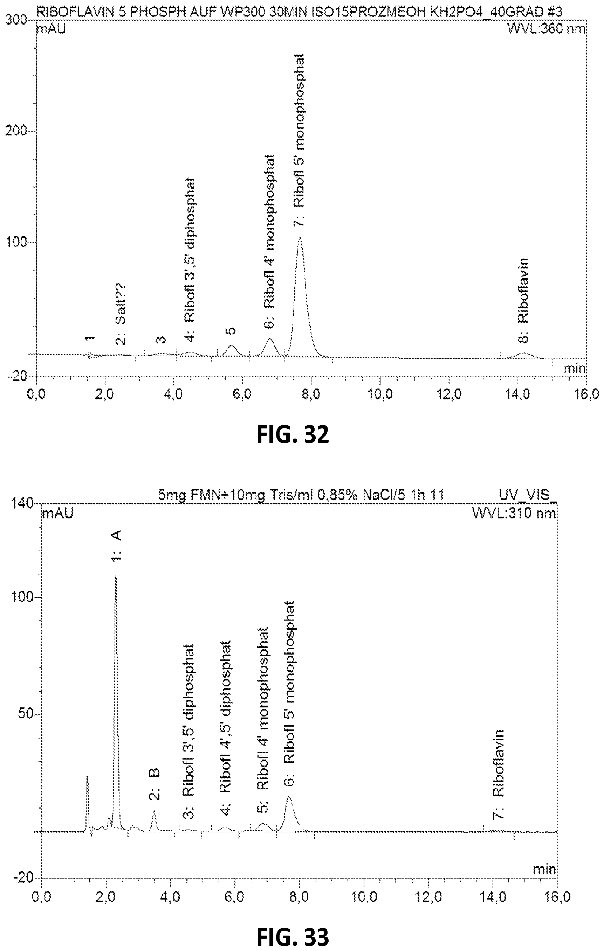

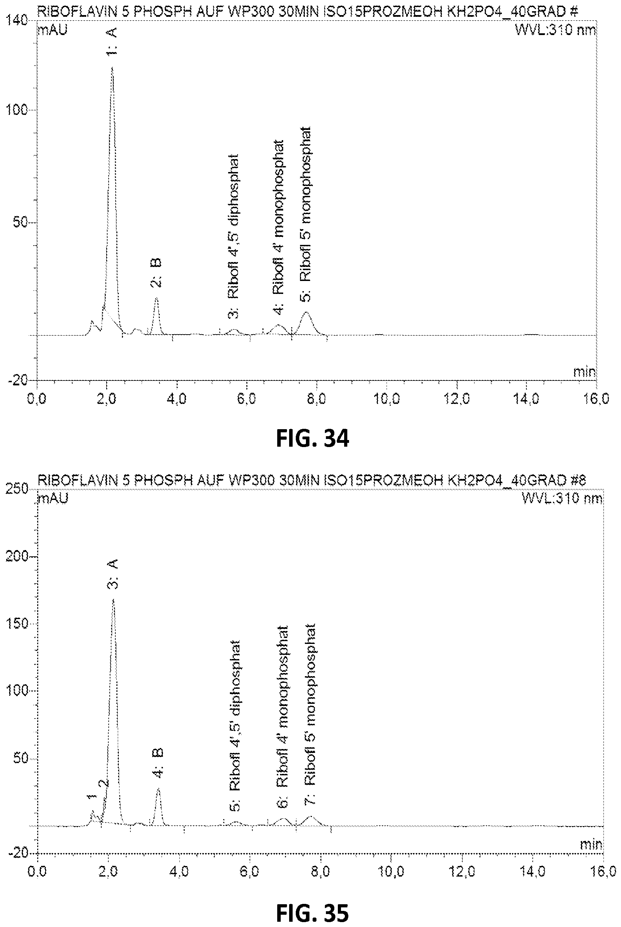

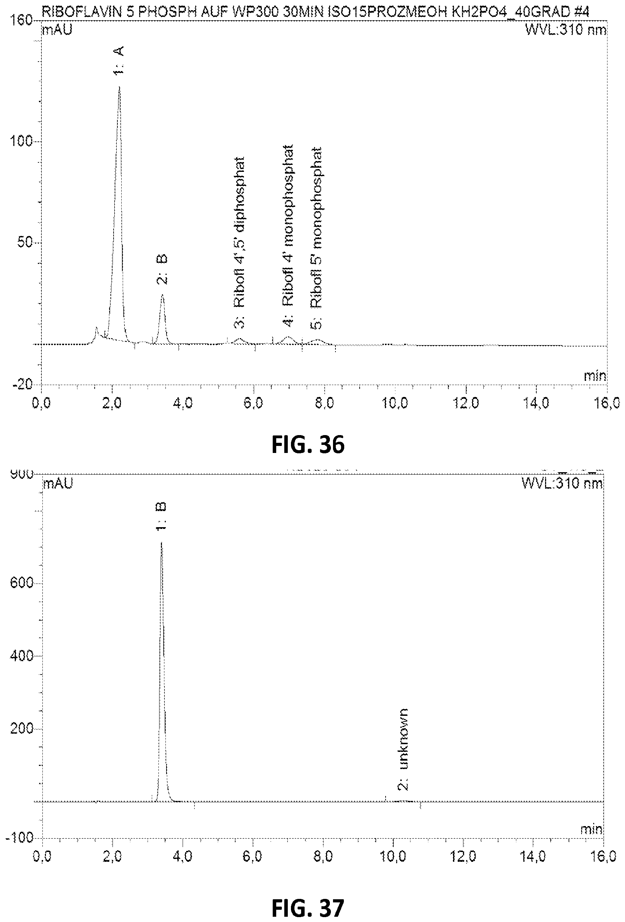

Degradation experiments proved that the synthesized compound 2 is also produced by thermal degradation of a monophosphorylated riboflavin (5-FMN) and corresponds to peak B in FIGS. 33 to 37. FIG. 32 illustrates monophosphorylated riboflavin (5-FMN) in buffered blood bank saline without thermal treatment. FIG. 33 illustrates 5-FMN in buffered blood bank saline after 1 hour of thermal treatment. FIG. 34 illustrates 5-FMN in buffered blood bank saline after 2 hours of thermal treatment. FIG. 35 illustrates 5-FMN in buffered blood bank saline after 3 hours of thermal treatment. FIG. 36 illustrates 5-FMN in buffered blood bank saline after 4 hours of thermal treatment. FIG. 37 illustrates HPLC trace of the synthesized riboflavin degradation product 2.

A second degradation product A is also produced during the thermal degradation of 5-FMN. Due to its more polar characteristics (shorter retention time) it is assumed that this peak corresponds to the phosphorylated quinoxaline compound. This assumption can be confirmed by comparing the UV spectra of both compounds. The similarity of the spectra indicates that no change at the chromophore has taken place. Therefore, it is assumed that this quinoxaline intermediate is formed by the loss of one molecule of urea without the hydrolysis of the phosphorous ester.

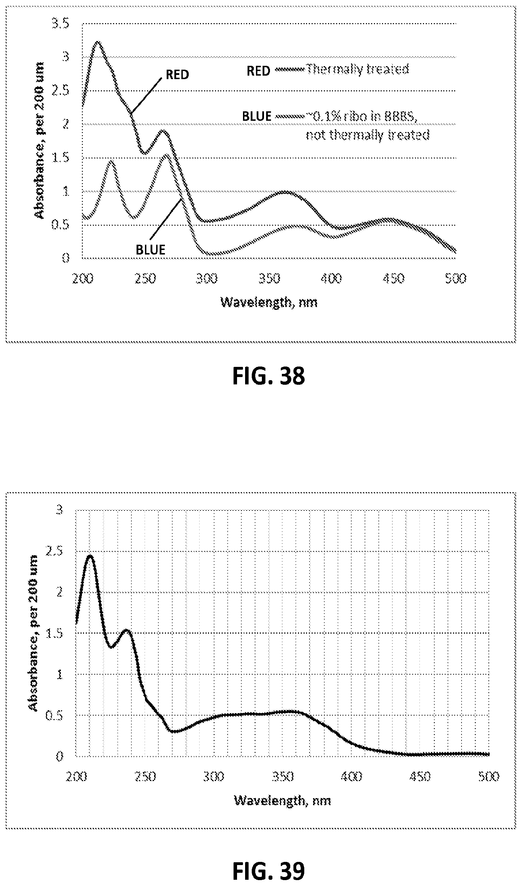

Riboflavin-5-phosphate solution (0.5% riboflavin) in 0.85% Blood Bank Buffered Saline (Thermo Scientific) was sealed in a plastic container and kept for 2 hours at 120 °C. Absorption of this solution was measured after the heat treatment and compared to the absorption of not treated solution (containing ∼0.1% riboflavin) in 0.85% BBBS (as shown in Figure 13) (pH of the solutions = 6.6 for thermally treated and 6.9 for not treated). FIG. 38 illustrates absorption spectra of thermally heated (red line) and not heated (blue line) riboflavin solutions (recorded in a quartz cuvette with 200 µm optical path). FIG. 39 illustrates the Difference between two spectra in FIG. 32.

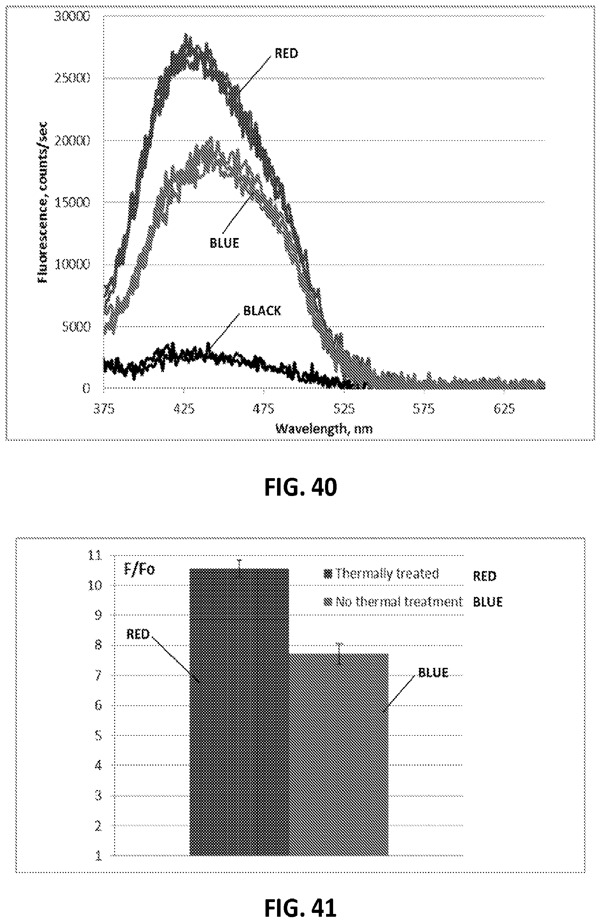

Two riboflavin solutions were used for cross-linking of porcine corneas (no epithelium, 20 min soak, 30 mW/cm² for 4 min continuous UVA exposure without application of riboflavin drops during the irradiation). Flaps were cut with the average thickness of 200 µm. Fluorescence of the digested with papain buffer corneas are presented on FIG. 40. In particular, FIG. 40 illustrates fluorescence of the digested corneas: non-cross-linked control (black line), cross-linked with 0.1% riboflavin which was not heated (blue line), cross-linked with thermally treated riboflavin solution (red line). FIG. 41 illustrates relative fluorescence of the cross-linked corneal samples: red - using thermally treated riboflavin solution, blue - using not heated 0.1% riboflavin solution.

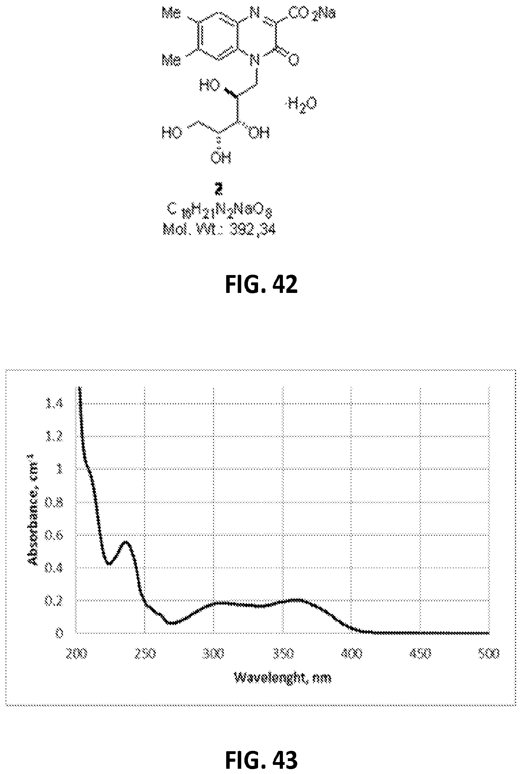

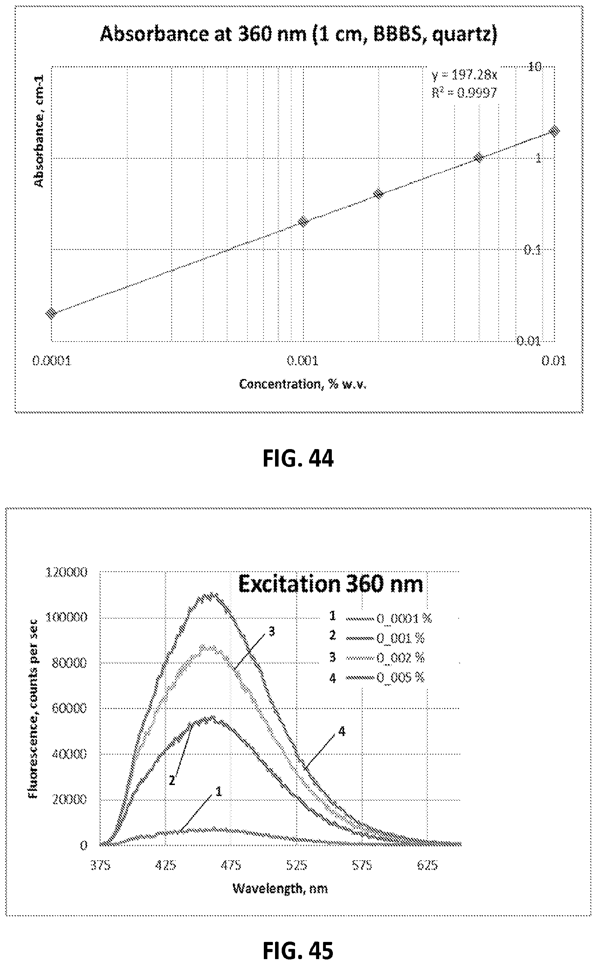

Sodium salt of 1,2-Dihydro-6,7-dimethyl-2-keto-1-D-ribityl-3-quinoxalinecarboxylic acid monohydrate, as shown in FIG. 42, has a strong absorbance at 360 nm (as shown in FIGS. 43 and 44) and noticeable fluorescence with a maximum around 460 nm (as shown in FIG. 45). FIG. 43 illustrates absorbance spectrum of 0.001% solution of sodium salt of 1,2-Dihydro-6,7-dimethyl-2-keto-1-D-ribityl-3-quinoxalinecarboxylic acid monohydrate in BBBS (quartz, 1 cm light path) FIG. 44 illustrates UV absorbance of sodium salt of 1,2-Dihydro-6,7-dimethyl-2-keto-1-D-ribityl-3-quinoxalinecarboxylic acid monohydrate at 360 nm (solution in BBBS, quartz cuvette with 1 cm light path). FIG. 45 illustrates fluorescence of sodium salt of 1,2-Dihydro-6,7-dimethyl-2-keto-1-D-ribityl-3-quinoxalinecarboxylic acid monohydrate in BBBS solutions (excitation 360 nm).

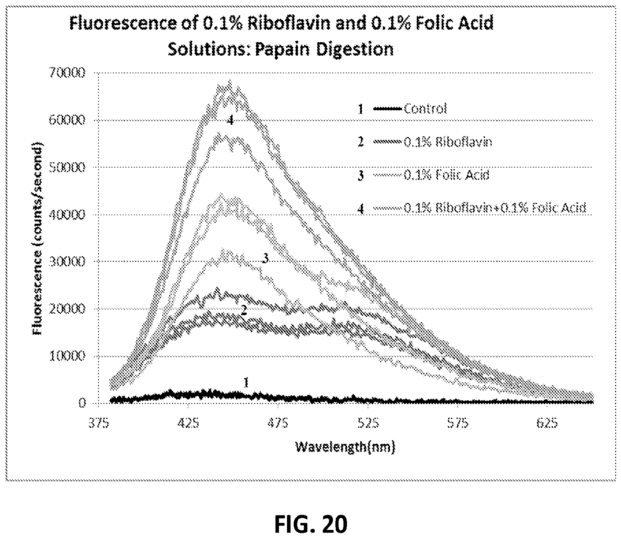

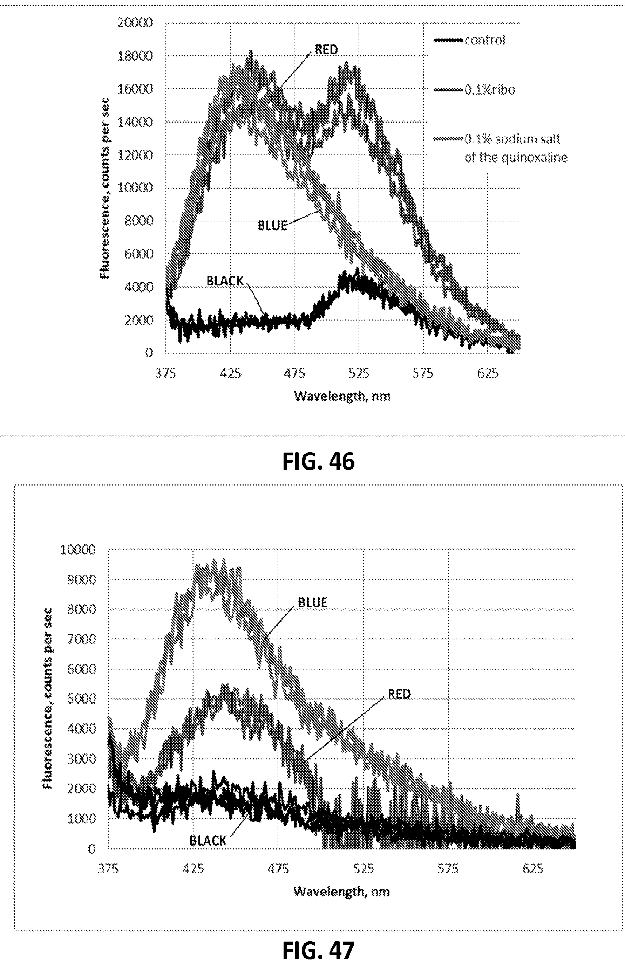

Solutions of 0.1% riboflavin-5-phosphate and 0.1% sodium salt of 1,2-Dihydro-6,7-dimethyl-2-keto-1-D-ribityl-3-quinoxalinecarboxylic acid monohydrate in BBBS were used for cross-linking of the porcine corneas (no epithelium, 20 min soak, 30 mW/cm² for 4 min continuous UVA exposure without application of riboflavin or other cross-linker drops during the irradiation). Flaps were cut with the average thickness of 200 µm. Fluorescence of the digested with papain buffer corneas are presented in Figure 20. Corneas cross-linked with riboflavin or sodium salt of 1,2-Dihydro-6,7-dimethyl-2-keto-1-D-ribityl-3-quinoxalinecarboxylic acid monohydrate both demonstrated elevated fluorescence in the area 450 nm (as shown in FIG. 46). FIG. 46 illustrates fluorescence of the digested corneas: non-cross-linked control (black line), cross-linked with 0.1% riboflavin in BBBS (red line), cross-linked with 0.1% sodium salt of 1,2-Dihydro-6,7-dimethyl-2-keto-1-D-ribityl-3-quinoxalinecarboxylic acid monohydrate (blue line).

In another experiment, 200 µm-thick corneal flaps without epithelium were cut off from the porcine eyes, placed in 1 mL 0.1% solution of riboflavin in BBBS or 0.1% solution of sodium salt of 1,2-Dihydro-6,7-dimethyl-2-keto-1-D-ribityl-3-quinoxalinecarboxylic acid monohydrate in BBBS, de-oxygenated by bubbling argon for 3 min, then sealed between quartz sheets and irradiated for 4 min at 30 mW/cm² in CO₂ atmosphere. Flaps were rinsed in distilled water, vacuum-dried and digested in the papain buffer. Fluorescence of the obtained solutions was recorded at excitation of 360 nm in order to evaluate collagen fluorescence (as shown in FIG. 47). FIG. 47 illustrates fluorescence of the digested corneal flaps: non-cross-linked control (black line), cross-linked with 0.1% riboflavin in BBBS (red line), cross-linked with 0.1% sodium salt of 1,2-Dihydro-6,7-dimethyl-2-keto-1-D-ribityl-3-quinoxalinecarboxylic acid monohydrate (blue line).

Fluorescence of the corneal flaps which were cross-linked with sodium salt of 1,2-Dihydro-6,7-dimethyl-2-keto-1-D-ribityl-3-quinoxalinecarboxylic acid monohydrate was higher than the fluorescence of the corneal flaps cross-linked with riboflavin. This suggests a lower sensitivity to the oxygen presence when sodium salt of 1,2-Dihydro-6,7-dimethyl-2-keto-1-D-ribityl-3-quinoxalinecarboxylic acid monohydrate is used as a cross-linking agent. Thus, according to aspects of the present disclosure, this salt may be employed in treatments where lower sensitivity to the presence of oxygen is advantageous.

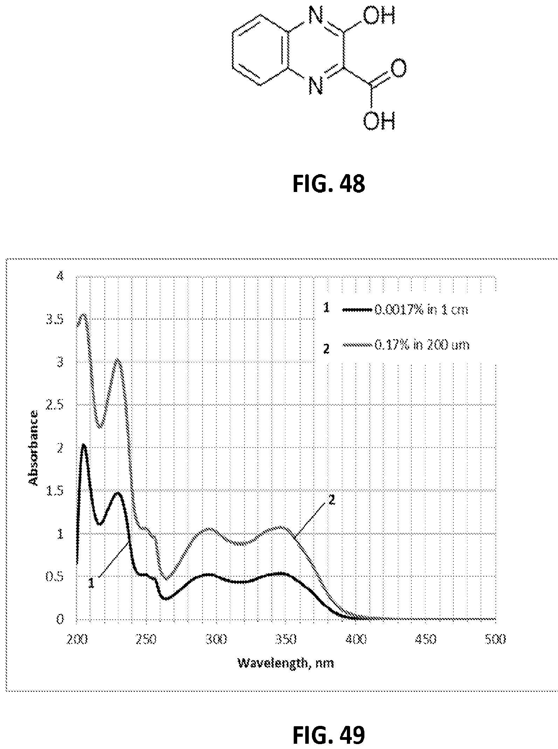

0.17% and 0.017% solutions of 3-hydroxy-2-quinoxalinecarboxylic acid, as shown in FIG. 48, were prepared in BBBS and recorded absorbance (in 200 µm and 1 cm - thick quartz quvettes) as presented in the FIG. 49. FIG. 49 illustrates absorbance spectra of 3-hydroxy-2-quinoxalinecarboxylic acid.

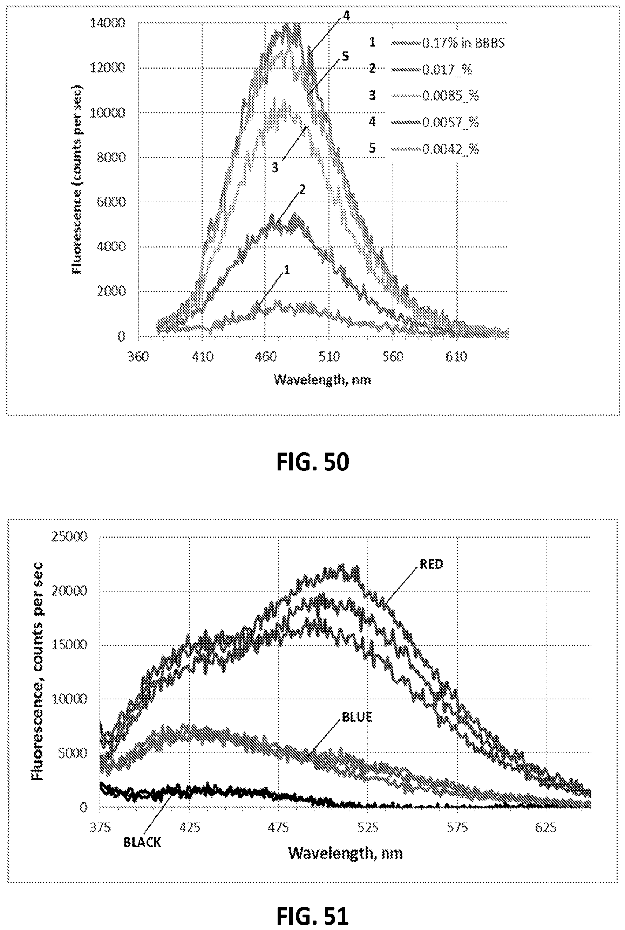

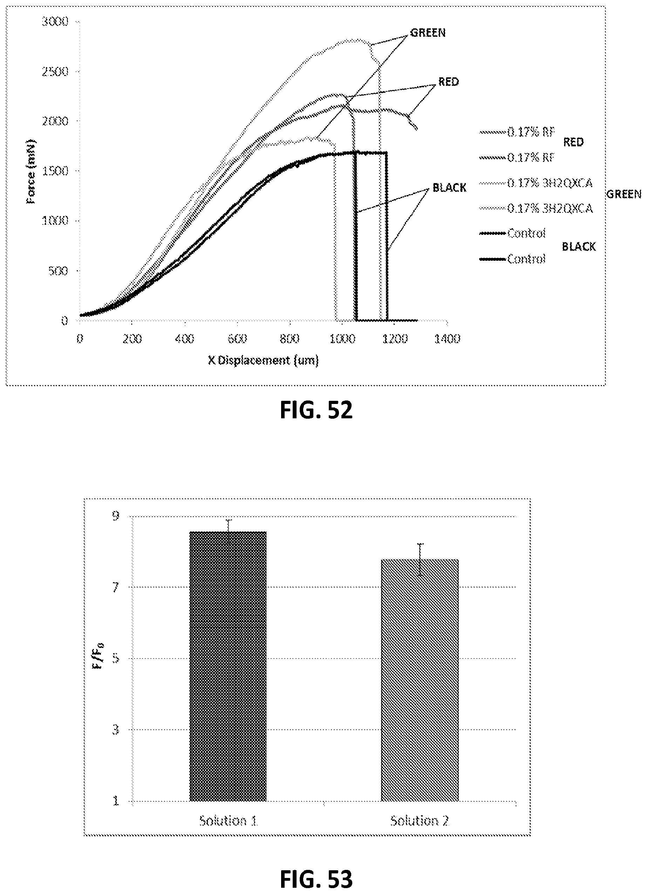

0.17% solution of 3-hydroxy-2-quinoxalinecarboxylic acid exhibited a strong fluorescence quenching (as shown in FIG. 50) because upon dilution, its fluorescence increased significantly. FIG. 50 illustrates fluorescence of 3-hydroxy-2-quinoxalinecarboxylic acid's solutions with different concentrations in BBBS, recorded with excitation of 360 nm. FIG. 51 illustrates fluorescence of the papain digested corneal flaps (200 µm thick) cross-linked with 0.17% (red lines) and 0.017% (blue lines) solutions of 3-hydroxy-2-quinoxalinecarboxylic acid in BBBS (no epithelium, 20 min soaking time, 30 mW/cm² for 4 min), relative to non-cross-linked controls (black lines). FIG. 52 illustrates tensiometry plots of 200- µm thick corneal flaps irradiated at 30 mW/cm2 for 4 min, preliminary saturated for 20 min with 0.17% riboflavin (red lines) and 0.17% 3-hydroxy-2-quinoxalinecarboxylic acid (3H2QXCA, green lines)relative to non-cross-linked controls (black lines). FIG. 53 illustrates relative fluorescence of the papain digested corneal flaps (200 µm thick) cross-linked with 0.1% riboflavin (blue bar, solution 2) and 0.1% riboflavin containing 0.02% solution of 3-hydroxy-2-quinoxalinecarboxylic acid in BBBS (red bar, solution 1) (no epithelium, 20 min soaking time, 30 mW/cm² for 4 min), where F₀ - fluorescence of a non-cross-linked flap.

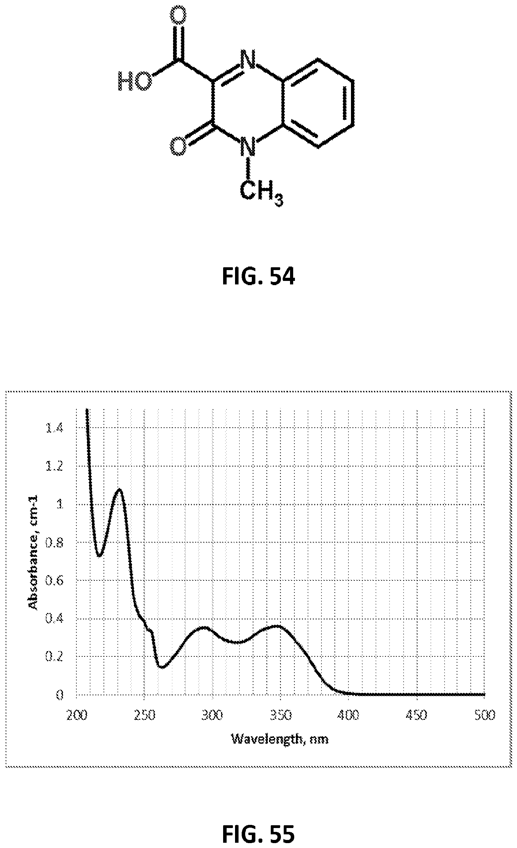

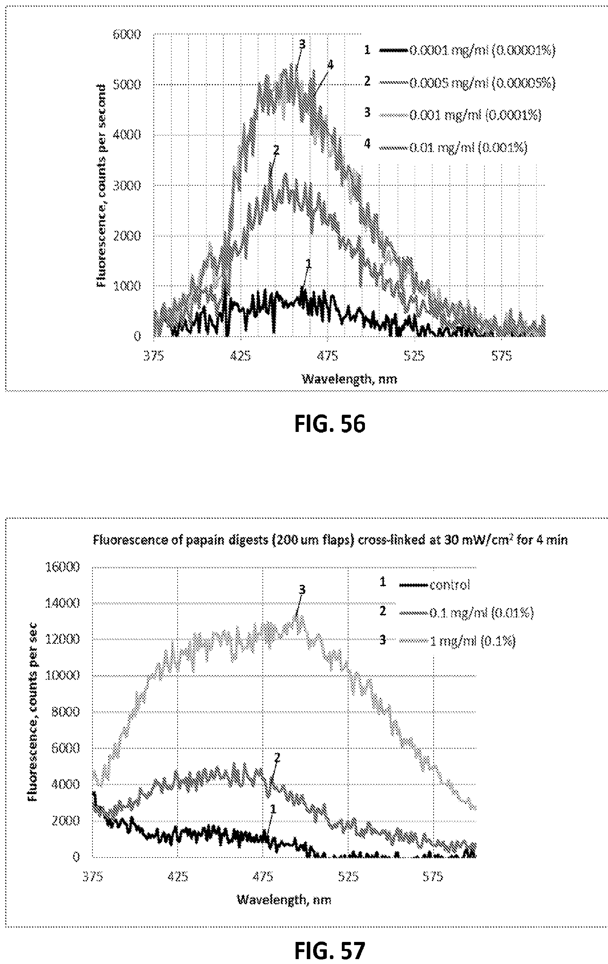

4-methyl-3-oxo-3,4-dihydro-2-quinoxaline carboxylic acid as shown in FIG. 54 has a strong absorbance at 360 nm (as shown in FIG. 55) and some fluorescence with a maximum around 460 nm (as shown in FIG. 56). FIG. 55 illustrates absorbance spectrum of 0.01 mg/ml solution of 4-methyl-3-oxo-3,4-dihydro-2-quinoxaline carboxylic acid in BBBS (quartz, 1 cm light path). FIG. 56 illustrates fluorescence of 4-methyl-3-oxo-3,4-dihydro-2-quinoxaline carboxylic acid in BBBS solutions (excitation 360 nm). FIG. 57 illustrates fluorescence of the papain-digested corneal flaps: non-cross-linked control (black line), cross-linked with 0.1 mg/ml (red line) and 1 mg/ml (green line) solutions of 4-methyl-3-oxo-3,4-dihydro-2-quinoxaline carboxylic acid in BBBS, where corneas were de-epithelialized, soaked with the solution of the cross-linker for 20 min and then irradiated for 4 min with 30 mW/cm² UVA light (360 nm).

According to aspects of the present disclosure, systems and methods for treating the eye employ cross-linking agents that are produced during the hydrolysis of riboflavin. For example, hydrolysis of riboflavin produces sodium salt of 1,2-Dihydro-6,7-dimethyl-2-keto-1-D-ribityl-3-quinoxalinecarboxylic acid monohydrate. It has been discovered that this salt is less susceptible to oxygen starvation (*i.e.,* lower sensitivity to oxygen presence) than conventional treatment solutions of riboflavin. When this salt is used alone or in combination with solutions of riboflavin, more effective cross-linking can be achieved for eye treatments.

According to aspects of the present disclosure, systems and methods employ thermal treatment of riboflavin solutions to achieve the results of the hydrolysis of riboflavin, including the production of cross-linking agents.

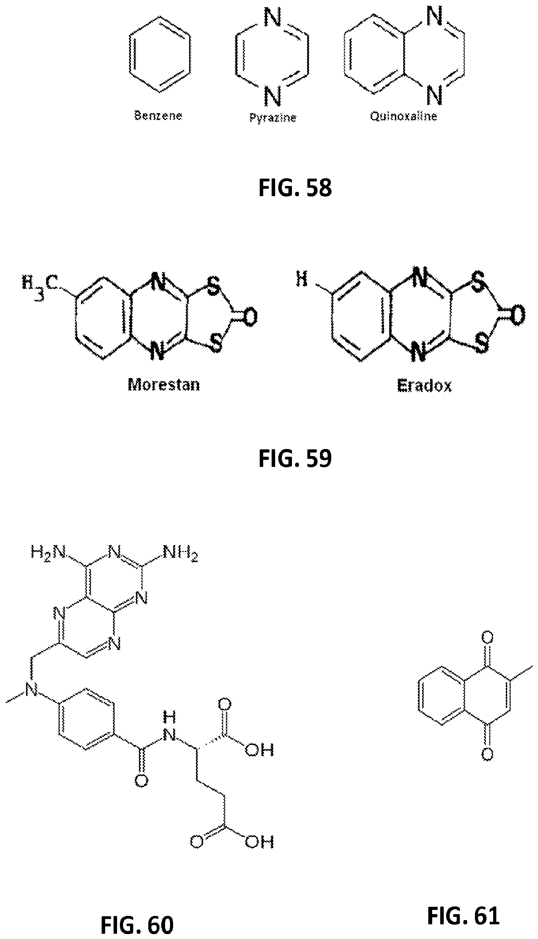

According to aspects of the present disclosure, systems and methods employ quinoxalines to cause cross-linking activity for eye treatments. Quinoxaline (also called benzopyrazine) is a heterocyclic compound containing a ring complex made up of benzene ring and a pyrazine ring as shown in FIG. 58. Quinoxaline derivatives are widely distributed in nature and many of them, such as the antibiotics, echinomycin, levomycin and actinoleutin possess very useful biological activity. In addition, a large number of synthetic quinoxalines have also shown antibacterial, fungicidal, insecticidal, anticancer, tranquilizing and antidepressant properties. According to aspects of the present disclosure, the ability of the quinoxalines to act as cross-linking agents may be employed with these other benefits, e.g., antibacterial or fungicidal characteristics, for certain eye treatments. The collaborative work by synthetic and screening research groups have continuously been carried out to create various biologically active quinoxalines. Thus quinoxaline 1,4-dioxides have been shown antibacterial and quinoxaline-2,3-dithione cyclic dithio-carbonate (Morestan) and trithiocarbonate (Eradox) (as shown in FIG. 59) possess fungicidal and insecticidal effects. The 2,3,7-trichloro-6-methylsulfamoyl quinoxaline has been patented as anticancer agent. 2-Phenyl-3-piperidino quinoxaline and some of its derivatives are selective herbicides.

### Cross-linking with Olaquindox (Reference example)

Olaquindox (N-(2-hydroxyethyl)-3-methyl-2-quinoxalinecarboxamide 1,4-dioxide) (VETRANAL™) has been used since 1975 as a growth promoter for farm animals because it has antibacterial characteristics. Olaquindox is related to the general class of quinoxalines. A commercially available preparation contains 10% olaquindox as an active ingredient in a calcium carbonate carrier together with 1.5% glyceryl polyethyleneglycol ricinoleate to reduce dust during preparation. The concentration of olaquindox in the feed is generally 50 ppm. Irradiated in the presence of human serum albumin, olaquindox disappears completely within 20 seconds; the N-monoxides are formed and a modified albumin which has altered properties in isoelectric focussing and electrophoresis systems. The following study evaluated the cross-linking potential of Olaquindox.

### A. Materials and Methods

Pig eyes were shipped overnight on ice from an abattoir (SiouxPreme, Sioux City, IA), rinsed in saline. Eyes were cleaned and epithelium was removed. Eyes were soaked for 20 minutes with 0.4% Olaquindox in PBS in an incubator set at 37°C by using a rubber ring to hold the solution on top. Some eyes were irradiated on air with continuous UVA light, and some placed in a beaker with a light oxygen stream for 2 minutes in the incubation chamber prior to irradiation. Corneas were pan-corneally irradiated with a top hat beam (3% root mean square) for 4 or 8 minutes with 365-nm light source (UV LED NCSU033B[T]; Nichia Co., Tokushima, Japan) at the chosen irradiance (30 mW/cm2, pulsed 1 sec on 1 sec off). UV irradiance was measured with a power sensor (model PD-300-UV; Ophir, Inc., Jerusalem, Israel) at the corneal surface. Corneal flaps (approximately 200 µm thick) were excised from the eyes with aid of Intralase femtosecond laser (Abbot Medical Optics, Santa Ana, CA). The average thickness of the corneal flaps was calculated as a difference between the measurements before and after the excision from the eyes with an ultrasonic Pachymeter (DGH Technology, Exton, PA. The flaps were placed into a biaxial extensometer (CellScale Biotester5000, Waterloo, ON), using biorake attachments with 5 tines spanning a width of 3 mm. Each sample was stretched at a constant rate of 4 µm/s in saline at 37°C until sample failure. The flaps were washed with distilled water 2 times, dried with filter paper, washed with dH₂O two times, and then dried in a vacuum until the weight change became less than 10% (Rotary vane vacuum pump RV3 A652-01-903, BOC Edwards, West Sussex, UK). Each flap was digested for 2.5 hours at 65°C with 2.5 units/ml of papain (from Papaya latex, Sigma) in 1 mL of papain buffer [BBBS (pH 7.0-7.2), 2 mM L-cysteine and 2 mM EDTA]. Papain digests were centrifuged for 5 seconds at 2200xG (Mini centrifuge 05-090-100, Fisher Scientific), diluted 0.5 times with 1XBBBS and fluorescence of the solutions was measured with excitation of λex = 360 nm in a QM-40 Spectrofluorometer (Photon Technology Int., London, Ontario, Canada). The fluorescence of the papain buffer was taken into account by measuring fluorescence in the absence of tissue and subtracting this value from the fluorescence of the samples.

### B. Results/Conclusion

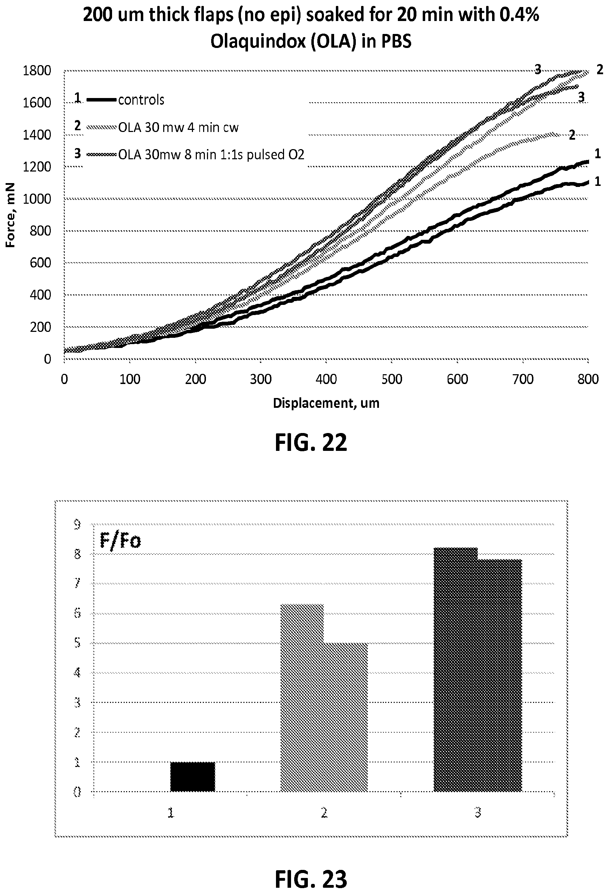

FIG. 22 illustrates biaxial extensiometry of 200 um thick corneal flaps, soaked with 0.4% Olaquindox in PBS with irradiation for 4 min with 30 mW/cm2 (CW) (2) and irradiation for 8 minutes (1 second on:1 second off) with 30 mW/cm2 of pulsed UVA light and O₂ (3), relative to controls (1).

FIG. 23 illustrates relative fluorescence recorded at 450 nm of the cross-linked flaps with 0.4% Olaquindox in PBS: (1) non-irradiated control; (2) irradiation for 4 min with 30 mW/cm² continuously (CW); and (3): irradiation for 8 minutes (1 second on:1 second off) with 30 mW/cm2 of pulsed UVA light and O₂.

After exposure with UVA, corneal collagen became stiffer and acquired fluorescence at 450 nm. As such, Olaquindox is an effective water-soluble cross-linking agent.

### Cross-linking with Riboflavin and Folic Acid (Reference example)

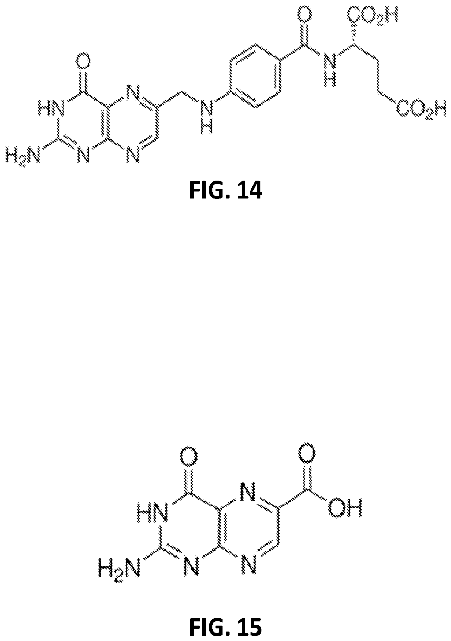

Folic acid (FA), or vitamin B₉, as shown in FIG. 14 is specifically considered by the United States Food and Drug Administration to be among safe medical food ingredients, under 21 CFR § 172.345(f). FA itself does not have a strong fluorescence and does not sensitize formation of singlet oxygen efficiently (most probably due to involvement of the p-aminobenzoylglutamate substituent in internal fluorescence quenching by radiationless deactivation of the singlet excited state leading to inefficient intersystem crossing). However, UV irradiation causes the oxidation of FA, leading to the formation of 6-formylpterin and then pterine-6-carboxylic acid (PCA) as shown in FIG. 15.

PCA is an efficient photosensitizer and generator of singlet oxygen. Therefore, both FA and PCA can generate collagen cross-linking. FA may be used in combination with riboflavin because addition of riboflavin markedly intensifies oxidation of FA while most of the riboflavin remains undecomposed.

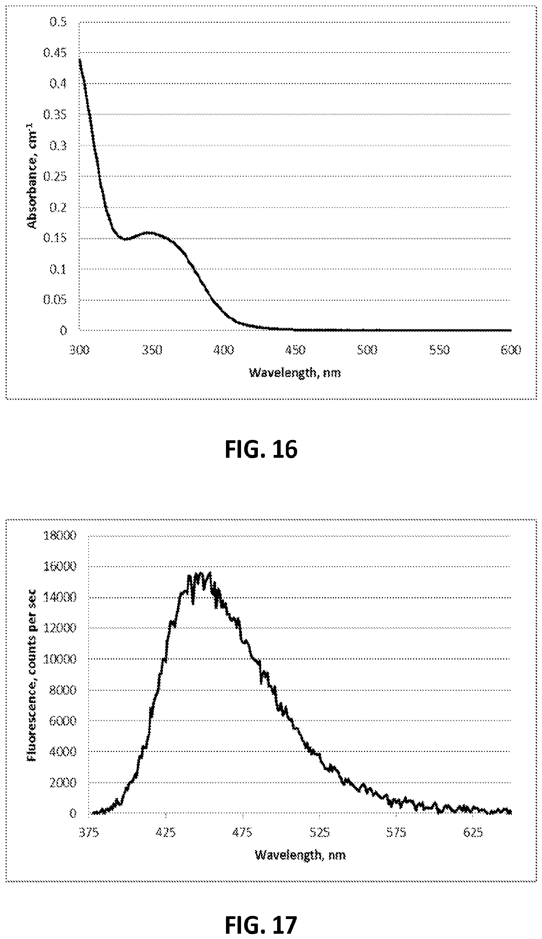

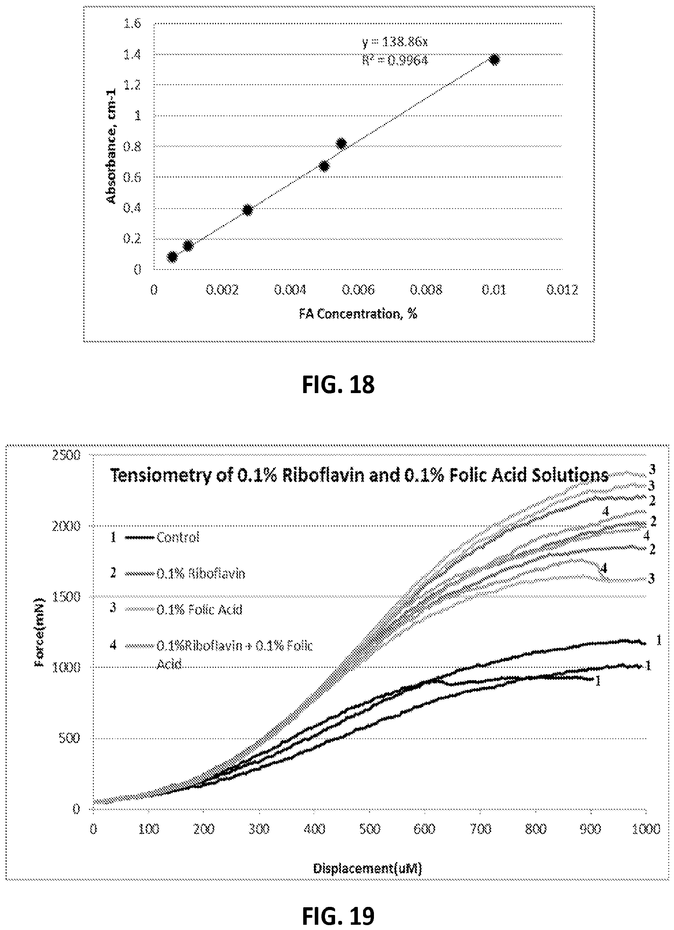

FA is soluble in water with dependence on pH and temperature. In the following studies, for example, its solubility in a phosphate buffer was 5.5 mg/ml at 25°C and pH of the final solution was 7.0. FA has UV light absorbance at 400 nm and below (the long wave peak at 360 nm as shown in FIG. 16), and fluorescence with maximum around 460 nm (as shown in FIG 17). FIG. 18 shows the absorbance of FA at 360 nm as a function of FA concentration in phosphate buffer.

### A. Materials and Methods

Pig eyes were shipped overnight on ice from an abattoir (SiouxPreme, Sioux City, IA), rinsed in saline. The eyes were cleaned and epithelium was removed. Sodium Phosphate Buffer (pH 7.6, made with Sodium Phosphate Monobasic, Sodium Phosphate Dibasic and Sodium Chloride in distilled water) was used as the buffer for all solutions. The final pH values for riboflavin solution, FA solution, and their mixture were in the range of 7.3-7.4. The eyes were soaked for 20 minutes with 0.1% FA, 0.1% Riboflavin, or 0.1% FA + 0.1% riboflavin in an incubator set at 37°C by using a rubber ring to hold the solution on top. The eyes were placed in a beaker filled with pure oxygen for 2 minutes in the incubation chamber prior to irradiation. Corneas were pan-corneally irradiated with a top hat beam (3% root mean square) for 8 minutes with 365-nm light source (UV LED NCSU033B[T]; Nichia Co., Tokushima, Japan) at the chosen irradiance (30 mW/cm², pulsed 1 second on: 1 second off) which was measured with a power sensor (model PD-300-UV; Ophir, Inc., Jerusalem, Israel) at the corneal surface. Corneal flaps (approximately 200 µm thick) were excised from the eyes with aid of Intralase femtosecond laser (Abbot Medical Optics, Santa Ana, CA). The average thickness of the corneal flaps was calculated as a difference between the measurements before and after the excision from the eyes with an ultrasonic Pachymeter (DGH Technology, Exton, PA). The flaps were placed into a biaxial extensometer (CellScale Biotester 5000, Waterloo, ON), using biorake attachments with 5 tines spanning a width of 3 mm. Each sample was stretched at a constant rate of 4 µm/s in saline at 37°C until sample failure. The flaps were washed with distilled water 2 times, dried with filter paper, washed with dH₂O two times, and then dried in a vacuum until the weight change became less than 10% (Rotary vane vacuum pump RV3 A652-01-903, BOC Edwards, West Sussex, UK). Each flap was digested for 2.5 hours at 65°C with 2.5 units/ml of papain (from Papaya latex, Sigma) in 1 ml of papain buffer [BBBS (pH 7.0-7.2), 2 mM L-cysteine and 2 mM EDTA]. Papain digests were centrifuged for 5 seconds at 2200xG (Mini centrifuge 05-090-100, Fisher Scientific), diluted 0.5 times with 1XBBBS and fluorescence of the solutions was measured with excitation of λex = 360 nm in a QM-40 Spectrofluorometer (Photon Technology Int., London, Ontario, Canada). The fluorescence of the papain buffer was taken into account by measuring fluorescence in the absence of tissue and subtracting this value from the fluorescence of the samples.

### B. Results/Conclusion

FIG. 19 illustrates displacement vs. force curves for corneal samples: (1) not exposed to UV light; (2) 0.1% riboflavin, exposed to UV light; (3) 0.1% FA, exposed to UV light; and (4) mixture of 0.1% riboflavin and 0.1% FA, exposed to UV light, with UV exposure of 365 nm, 30 mW/cm², pulsed 1 second on: 1 second off for 8 minutes total, with oxygen ambience over the cornea.

FIG. 20 illustrates fluorescence of the corneal samples after digestion with papain (excitation 360 nm): (1) not exposed to UV light; (2) 0.1% riboflavin, exposed to UV light; (3) 0.1% FA, exposed to UV light; and (4) mixture of 0.1% riboflavin and 0.1% FA, exposed to UV light, with UV exposure at 365 nm, 30 mW/cm², pulsed 1 second on: 1 second off for 8 minutes total, with oxygen ambience over the cornea.

As shown in FIG. 19, the effect of the exposure of UV light on corneal collagen is very similar for all three groups of the studied solutions (0.1% riboflavin, 0.1% FA, and mixture of 0.1% riboflavin with 0.1% FA). As compared to the unexposed controls, soaking with a solution and then exposing it to UV leads to significant stiffening of a corneal sample. FIG. 20 shows that fluorescence of the cross-linked collagen samples increases as compared to the non-exposed to UV control samples.

As described above, it is also contemplated that FA can also be used as a primary cross-linking agent (without riboflavin).

### C. Additional Experiment (Reference example)

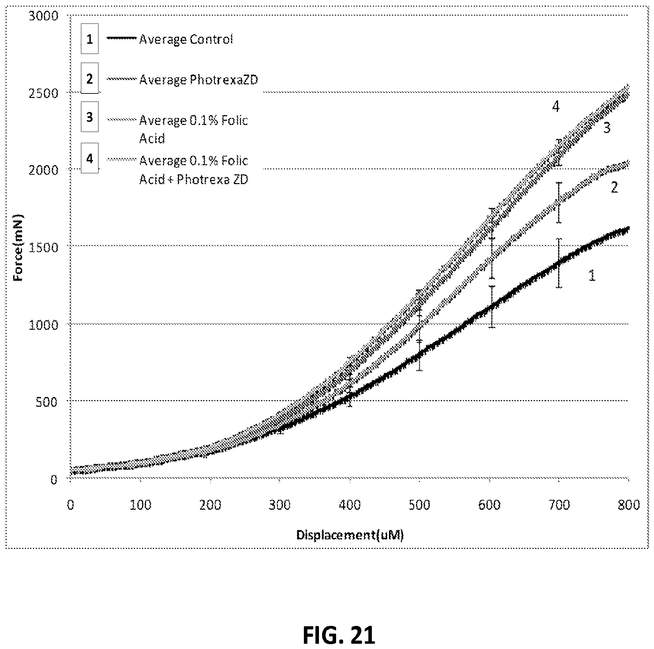

An additional experiment was conducted when FA was dissolved in a formulation solution containing 0.1% riboflavin in buffer saline solution (available under AVEDRO® PHOTREXA ZD®). FIG. 21 illustrates displacement vs. force curves for corneal samples (thickness 300 um, 3 samples in each group): (1) controls unexposed to UV light; (2) 0.1% FA in a buffer, exposed UV light; (3) 0.1% riboflavin in buffer saline solution, exposed to UV light; and (4) mixture of 0.1% FA in 0.1% riboflavin in buffer saline solution, exposed to UV light, UV exposure at 365 nm, 30 mW/cm², pulsed 1 second on: 1 second off for 8 minutes total, with oxygen ambience over the cornea.

### Other Cross-Linking Agents

The drugs chloroquine, hydroxychloroquine, quinine, and dibucaine may possess photosensitizing capability in aqueous solutions, e.g., by irradiation with 365 nm UV light. Chloroquine, hydroxychloroquine, and quinine are related to the general class of quinolines. According to aspects of the present disclosure, these drugs, based on their photosensitizing capability, may be applied as cross-linking agents in treatments of the cornea.

Methotrexate, as shown in FIG. 60, is a generic name of an immunosuppressive medication which has been used for treatment of certain cancers, and inflammatory diseases such as rheumatoid arthritis and uveitis. Similarly to FA, MTX has a significant UV-light absorbance at 360 nm. An idea of using MTX as a possible collagen cross-linker comes from the data about MTX photosensitizing properties measured on rabbit eyes conjunctive.

Pronounced UVA-photosensitization of thymidine has been observed with menadione (vitamin K₃), as shown in FIG. 61, and formation of the photo-oxidation product 5,6-dihydroxy-5,6-dihydrothymidine occurs after single electron transfer reactions between triplet state menadione and thymidine. Menadione can be a UVA photosensitizer, and therefore, a cross-linking agent for collagen.

Accordingly, various agents and additives for cross-linking treatments are identified and described in studies. The characteristics of the various agents and additives may be advantageously employed in formulations applied in cross-linking treatments of the eye. In the present invention, riboflavin is combined with Iron(II) to enhance the cross-linking activity generated by the riboflavin. In other aspects of the disclosure cross-linking treatments employ an Iron(II) solution in combination with a hydrogen peroxide pre-soak. In yet other aspects of the disclosure, 2,3-butanedione is employed to increase the efficacy of corneal cross-linking with a photosensitizer, such as riboflavin. In further aspects, folic acid is employed in combination with a photosensitizer, such as riboflavin, to enhance cross-linking activity. In yet further aspects, 2,3-butanedione, folic acid, a quinoxaline, a quinoline, dibucaine, Methotrexate, menadione, or a derivative thereof is applied as a cross-linking agent.

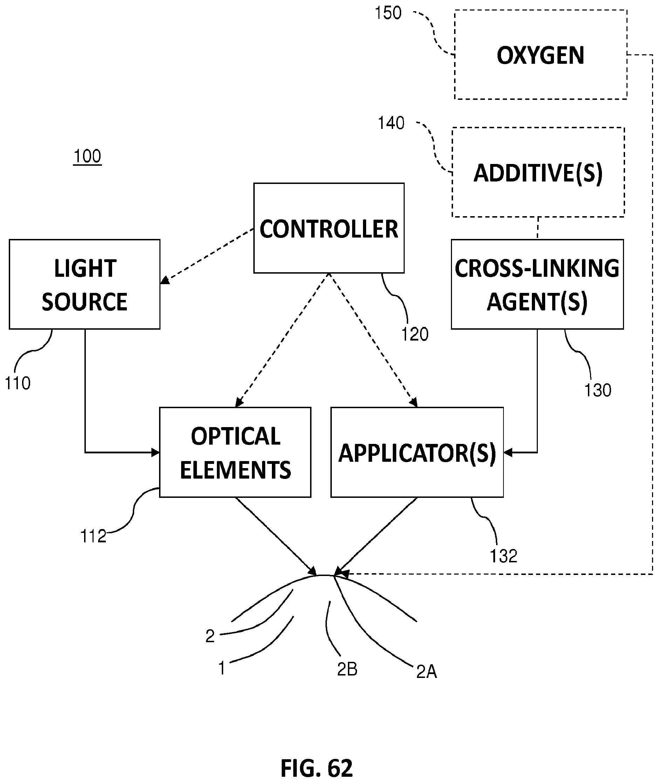

FIG. 62 provides a block diagram of an example delivery system 100 for delivering cross-linking agent(s) 130 (optionally combined with additive(s) 140) and photo-activating light to a cornea 2 of an eye 1 in order to initiate cross-linking of corneal collagen within the cornea 2. The delivery system 100 includes one or more applicators 132 for applying the cross-linking agent(s) 130 to the cornea 2. The cross-linking agent(s) 130 may include any of the photosensitizers described above. In addition, the cross-linking agent(s) 130 may be combined with any of the additives 140 described above to enhance the cross-linking activity generated by the cross-inking agent. In some cases, as described above, the additives described above can as primary cross-linking agents, and vice versa. The one or more applicators 132, for example, may include an eye dropper, syringe, or the like for applying the cross-linking agent(s) 130 in a solution. The one or more applicators 132 may apply the cross-linking agent(s) 130 according to a particular pattern on the cornea 2 where cross-linking activity may be more advantageous. The delivery system 100 may also deliver oxygen 150 to the cornea to control the cross-linking activity further.

The delivery system 100 includes a light source 110 and optical elements 112 for directing the photo-activating light to the cornea 2. The optical elements 112 may include, for example, one or more mirrors or lenses for directing and focusing the photo-activating light emitted by the light source 110 according to a particular pattern on the cornea 2 suitable for activating the cross-linking agent(s) 130. The light source 110 may be an ultraviolet (UV) light source, and the photo-activating light directed to the cornea 2 through the optical elements 112 activates the cross-linking agent(s) 130. The light source 110 may also alternatively or additionally emit photons with greater or lesser energy levels than UV light photons. The optical elements 112 can be used to focus the light emitted by the light source 110 to a particular focal plane within the cornea 2, such as a focal plane that includes a mid-depth region 2B. In addition, according to particular embodiments, the optical elements 112 may include one or more beam splitters for dividing a beam of light emitted by the light source 110, and may include one or more heat sinks for absorbing light emitted by the light source 110. The optical elements 112 may further include filters for partially blocking wavelengths of light emitted by the light source 110 and for advantageously selecting particular wavelengths of light to be directed to the cornea 2 for activating the cross-linking agent(s) 130.

The delivery system 100 also includes a controller 120 that may be coupled to the one or more applicators 132, the light source 110, and/or the optical elements 112. By controlling aspects of the operation of the one or more applicators 132, the light source 110, and/or the optical elements 112, the controller 120 can control the regions of the cornea 2 that receive the cross-linking agent(s) 130 and/or that are exposed to the light source 110. As such, the controller 120 can control the particular regions of the cornea 2 that are strengthened and stabilized through cross-linking of the corneal collagen fibrils. In an implementation, the cross-linking agent(s) 130 can be applied generally to the eye 1, without regard to a particular region of the cornea 2 requiring strengthening, but the light source 110 can be selectively directed to particular regions of the cornea 2 requiring strengthening, and thereby control the region of the cornea 2 wherein cross-linking is initiated by controlling the regions of the cornea 2 that are exposed to the light source 110. To control with precision the delivery of the light from the light source 110 to the cornea 2, the controller 120 may control any combination of: wavelength, bandwidth, intensity, power, location, depth of penetration, and duration of treatment. The controller 120 may include hardware and/or software elements, and may be a computing device. The controller 120 may include a processor, memory storage, a microcontroller, digital logic elements, software running on a computer processor, or any combination thereof. In an alternative implementation of the delivery system 100, the controller 120 may be replaced by two or more separate controllers or processors. In addition, the function of the controller 120 can be partially or wholly replaced by a manual operation. For example, the applicator 132 can be manually operated to deliver the cross-linking agent(s) 130 to the cornea 2 without the assistance of the controller 120. In addition, the controller 120 can operate the applicator 132 and/or the optical elements 112 according to inputs dynamically supplied by an operator of the delivery system 100 in real time, or can operate according to a pre-programmed sequence or routine.

## Claims

1. A composition for use in applying an enhanced cross-linking therapy to a cornea of an eye, comprising:
a cross-linking agent which is riboflavin, the cross-linking agent generating cross-linking activity in the cornea in response to exposure to a photo-activating light; and
an additive different from the cross-linking agent and including iron to enhance the cross-linking activity generated by the cross-linking agent.

2. The composition for use of claim 1, wherein the iron is provided by FeSO₄.

3. The composition for use of claim 1, wherein the additive enhances the cross-linking activity generated by the riboflavin by accelerating decomposition of hydrogen peroxide during photo-activation of the riboflavin and to increase a concentration of hydroxyl radicals.

## Patentansprüche

1. Zusammensetzung zur Verwendung bei der Anwendung einer Vernetzungserhöhungstherapie auf die Hornhaut eines Auges, die Folgendes umfasst:
einen Vernetzer, der Riboflavin ist, wobei der Vernetzer als Reaktion auf Bestrahlung mit photoaktivierendem Licht Vernetzungsaktivität in der Hornhaut erzeugt; und
ein Additiv, das sich vom Vernetzer unterscheidet und Eisen umfasst, um die durch den Vernetzer erzeugte Vernetzungsaktivität zu erhöhung.

2. Zusammensetzung zur Verwendung nach Anspruch 1, wobei das Eisen von FeSO₄ bereitgestellt wird.

3. Zusammensetzung zur Verwendung nach Anspruch 1, wobei das Additiv die durch das Riboflavin erzeugte Vernetzungsaktivität erhöht, indem die Zersetzung von Wasserstoffperoxid während der Photoaktivierung von Riboflavin beschleunigt wird und die Konzentration von Hydroxylresten erhöht wird.

## Revendications

1. Composition à utiliser pour appliquer une thérapie de réticulation améliorée à une cornée d'un œil, comprenant :
un agent de réticulation qui est la riboflavine, l'agent de réticulation générant une activité de réticulation dans la cornée en réponse à une exposition à une lumière photo-activatrice ; et
un additif différent de l'agent de réticulation et comprenant du fer pour améliorer l'activité de réticulation générée par l'agent de réticulation.

2. Composition à utiliser selon la revendication 1, dans laquelle le fer est fourni par FeSO₄.

3. Composition à utiliser selon la revendication 1, dans laquelle l'additif améliore l'activité de réticulation générée par la riboflavine en accélérant la décomposition de peroxyde d'hydrogène pendant une photo-activation de la riboflavine et en augmentant une concentration de radicaux hydroxyles.
